# EUROPEAN PATENT SPECIFICATION

(11) **EP 4 663 224 B1**
(45) Date of publication and mention of the grant of the patent: **25.11.2026**
(21) Application number: 25197707.0
(22) Date of filing: 08.07.2020
(51) Int. Cl.: A61M 16/08, A61M 16/16, A61M 39/10, A61M 16/00, A61M 16/10

(54) **MEDICAL TUBES AND CONNECTORS FOR BREATHING CIRCUITS**
MEDIZINISCHE SCHLÄUCHE UND VERBINDER FÜR BEATMUNGSKREISLÄUFE
TUBES MÉDICAUX ET CONNECTEURS POUR CIRCUITS RESPIRATOIRES

(30) Priority: 08.07.2019 US 201962871668 P
(43) Date of publication of application: 17.12.2025
(62) Document number(s) of the earlier application(s) in accordance with Art. 76 EPC: 20837062.7 / 3 996 788
(73) Proprietor: Fisher & Paykel Healthcare Limited, Auckland, 2013 (NZ)
(72) Inventor: HOLYOAKE, Bruce Gordon, Auckland, 2013 (NZ); VAN BOKHOVEN, David, Auckland, 2013 (NZ); NASIMI, Emma Louise, Auckland, 2013 (NZ); DEVERICK, Eric, Auckland, 2013 (NZ); EDMONDS, Ieuan Gregory Nicholas, Auckland, 2013 (NZ); GULLIVER, Laurence, Auckland, 2013 (NZ); BRAND, Levi Jard, Auckland, 2013 (NZ); ANDRESEN, Michael John, Auckland, 2013 (NZ); MOSEN, Rachel Nicole, Auckland, 2013 (NZ); BHONGALE, Sanket Raghunath, Auckland, 2013 (NZ); OU, Yangda, Auckland, 2013 (NZ); MARINOVICH, Matthew Michael, Auckland, 2013 (NZ)
(74) Representative: Lewis Silkin LLP

(56) References cited:
- WO-A1-2004/108218
- WO-A1-2015/093989
- WO-A1-2016/207838
- US-A- 5 368 017
- US-A1- 2015 101 600
- US-A1- 2017 197 055

## Description

### FIELD OF THE INVENTION

The present technology relates to tubes and connectors for medical use, and particularly medical tube assemblies, medical tubes and connectors for use in breathing circuits suitable for delivering humidified gases to or from a patient, such as in respiratory humidification systems.

### BACKGROUND

In breathing circuits, various components transport warm and/or humidified gases to and from patients. Respiratory humidification helps reduce the likelihood of infection and/or tissue damage. Examples of devices known in the art include those disclosed by US2017/197055A1, US2015/101600A1, WO2016/207838A1, WO2004/108218A1 and US5368017A.

Humidifiers are used to provide humidification to the gases. Medical tubes are used to transport the humidified gases to and from a patient and to connect together any devices as part of a breathing circuit.

A breathing circuit may provide for a complete circuit of breathing gases to and from the patient. In some cases no tube is provided to remove gases from the patient and gas can be exhaled directly to atmosphere. In other cases a full breathing circuit is provided to deliver gases to a patient as well as to remove them.

It is an object of the present technology to provide a medical tube assembly, and/or medical tube, and/or medical connector, or at least provide the public with a useful choice.

### SUMMARY

The invention is defined by the appended claims.

Disclosed is a medical tube assembly, medical tube and/or connector, for use with a respiratory apparatus and a breathing circuit. The configuration of the invention is defined by the appended claims, while the present disclosure contains many examples relevant, but not necessarily within, the scope of the claimed invention.

In an aspect there may be provided a medical tube assembly, the medical tube assembly comprising:
a tube, the tube comprising:
   a lumen extending from a first end of the tube to a second end of the tube,
   a first elongate member comprising a hollow body spirally wound to form at least in part said lumen,
   a second elongate member spirally wound and joined between adjacent turns of the first elongate member, and
   wherein a first end of the tube comprises at least one connector.

The second elongate member may form at least a portion of the lumen.

The first elongate member of the tube may form in longitudinal cross-section a plurality of hollow portions each with a flattened surface forming at least part of the wall surrounding the lumen.

The medical tube may comprise at least one wire.

The wire may be located between the plurality of hollow portions and the lumen of the tube.

The wire may be located within the second elongate member.

The lumen may be formed by the first elongate member and the second elongate member comprises a substantially smooth bore.

In another aspect there may be provided medical tube assembly, the medical tube assembly comprising:
a tube, the tube having a lumen extending from a first end of the tube to a second end of the tube, and
wherein a first end of the tube comprises at least one connector
wherein the lumen of the tube has a substantially smooth bore.

The medical tube may comprise at least one wire.

An outer surface of the tube may be corrugated, and optionally the corrugations are annular or helical.

The at least one wire may extend along a portion of the length of the medical tube.

The at least one wire may comprise at least one heater wire.

The at least one wire may comprise a pair of heater wires.

The at least one wire may comprise at least one sensor wire.

The at least one wire may comprise a pair of sensor wires.

A or the heating wire may be located within the lumen of the medical tube.

A or the heating wire may be attached to an inner wall of the medical tube.

The heating wire may be embedded within a wall of said medical tube.

A wire may be embedded within a second elongate member.

An internal diameter of the lumen of the medical tube may be about 10 mm to about 25 mm, or about 13 mm to about 20 mm, or about 16 mm to about 18 mm, or about 17.

A length of the medical tube may be about 1.4 m to about 1.8 m or about 1.5 m to about 1.7 m.

The medical tube may be a respiratory tube.

The medical tube may be an inspiratory tube

In another aspect there may be provided a medical tube assembly, the medical tube assembly comprising:
a medical tube, the medical tube having at least one wire located in a tube wall of the medical tube,
a connector, the connector located at an end of the medical tube.
wherein the connector comprises at least one electrical port, the electrical port configured to provide an electrical connection with said at least one wire, optionally the electrical port comprises at least connection point, the connection point configured to provide for connection of the electrical port to the at least one wire.

The at least one wire may be spirally wound along at least a portion of the length of the medical tube.

In another aspect there may be provided medical tube assembly, the medical tube assembly comprising:
a medical tube, the medical tube having at least one wire located in a tube wall of the medical tube, wherein the wire is helically wound,
a connector, the connector located at an end of the medical tube,
wherein the connector comprises at least one electrical port, the electrical port extending outwardly from the connector, the electrical port configured to provide an electrical connection with said at least one wire, optionally the electrical port comprises at least connection point, the connection point configured to provide for connection of the electrical port to the at least one wire,
wherein the connector comprises at least one guide feature configured to direct the at least one wire from the tube wall of the tube, and/or a pin connected to said at least one wire, to the electrical port and/or the connection point of the electrical port,
wherein the electrical port is located tangentially with respect to an outer surface of the connector.

In another there may be provided a connector for a medical tube, wherein the connector comprises:
a first portion having a first opening configured to receive a flow of gases,
a second portion having a second opening, the second portion configured to engage with a medical tube and provide a flow of gases to the medical tube through the second opening,
a lumen formed by the first portion and the second portion, the lumen providing a gases flow path between the first opening and the second opening,
wherein the first portion of the connector comprises at least one sensor port, the sensor port extending, through a wall of the first portion of the connector into the lumen,
wherein the second portion of the connector body comprises at least one electrical port, the electrical port configured to provide for electrical connection with at least one wire of the medical tube.

In another aspect there may be provided a connector for a medical tube, wherein the connector comprises:
a first portion having a first opening configured to receive a flow of gases,
a second portion having a second opening, the second portion configured to engage with a medical tube and provide a flow of gases to the medical tube through the second opening,
a lumen formed by the first portion and the second portion, the lumen providing a gases flow path between the first opening and the second opening,
wherein the first portion of the connector comprises at least one sensor port, the sensor port extending, through a wall of the first portion of the connector into the lumen,
wherein the second portion of the connector body comprises at least one electrical port, the electrical port configured to provide for electrical connection with at least one wire of the medical tube, and wherein the electrical port is located on the top of the second portion of the connector,
wherein the first portion is angled with respect to the second portion to form an elbow.

The medical tube may be defined by either or any combination of any of the other aspects.

The connector may comprise:
a first portion having a first opening configured to receive a flow of gases,
a second portion having a second opening, the second portion configured to engage with a medical tube and provide a flow of gases to the medical tube through the second opening,

A lumen may be formed by the first portion and the second portion, the lumen providing a gases flow path between the first opening and the second opening.

The first portion may be angled with respect to the second portion to form an elbow.

The angle between the first portion and the second portion may be about 90 degrees to about 170 degrees, or about 100 degrees to about 150 degrees, or about 110 to about 140 degrees, or about 135 degrees.

The first portion may provide for a taper fit connection.

The first portion of the connector may comprise a sensor port, the sensor port configured to receive a sensor.

The sensor port may provide an aperture for the sensor to be inserted so as to be in contact with a flow of gases in the first portion of the connector.

The sensor may be configured to measure flow rate of the flow of gases in the first portion of the connector.

The sensor may be configured to measure temperature of the flow of gases in the first portion of the connector.

The sensor may be configured to measure humidity of the flow of gases in the first portion of the connector.

Said sensor may be comprised as part of a probe, the probe for being received by the sensor port.

The second portion of the connector may comprise at least one electrical port, the electrical port configured to provide an electrical connection to the or at least one wire in the tube.

The sensor port may be located on a side of the first portion of the connector and the electrical port is located on the top of the second portion of the connector.

The sensor port may be offset about 120 to about 75 degrees, or about 110 to about 80 degrees, or about 90 degrees, about a longitudinal axis of the lumen of the first portion relative to electrical port.

The sensor port may be offset about 120 to about 75 degrees, or about 110 to about 80 degrees, or about 90 degrees, about a longitudinal axis of the lumen relative to electrical port.

Opposite sides of the elbow may define internal and external elbow surfaces, and wherein the electrical port is located on the external elbow surface.

The sensor port may be oriented perpendicular to the first portion.

The sensor port may be oriented perpendicular to a longitudinal axis of the first portion.

The electrical port may be oriented along an axis substantially parallel to an axis substantially perpendicular to the second portion.

The electrical port may be oriented along an axis substantially parallel to an axis perpendicular to a longitudinal axis of the second portion.

The electrical port may be offset from a longitudinal central axis of the second portion of the connector.

The sensor port may have a longitudinal axis, the sensor port allowing communication with an interior of the connector

The longitudinal axis of the sensor port may be oriented substantially perpendicularly to the flow of gases through the first portion of the connector.

The longitudinal axis of the sensor port may substantially intersect a corresponding longitudinal axis of the first portion of the connector from which the sensor port depends.

The sensor port may comprise a substantially cylindrical cross-section.

A terminal surface of the sensor port distal of an outer surface of the first portion may comprise one or more locating features.

The one or more locating features may comprise one or more notches of the terminal surface of the sensor port.

The one or more notches may extend in the direction of the longitudinal axis of the sensor port.

The connector may comprise a grommet, the grommet configured to be located with the sensor port.

The grommet may be integrally formed with the sensor port, and/or a jacket.

The locating features may comprise a plurality of notches, the notches spaced apart about a perimeter of the terminal surface and at least one of the plurality of notches configured to mate with a corresponding number of projections of the grommet.

The grommet may comprise a sensor orienting notch for receiving a projection of a or the sensor and rotationally orienting said sensor about the longitudinal axis of the sensor port.

The sensor port may comprise a sensor orienting notch for receiving a projection of a or the sensor and rotationally orienting said sensor about the longitudinal axis of the sensor port.

The grommet may comprise a sensor locating feature for engaging with a surface of a sensor and locating said sensor at a desired location within the sensor port.

The sensor locating feature may comprise a radially extending lip spaced away from the terminal surface of the sensor port and projecting inwardly towards the longitudinal axis of the sensor port.

The connector may comprise a jacket for the sensor port, the jacket for fitment at least partially about the sensor port, and/or the grommet.

The jacket may comprise a sensor orienting notch for receiving a projection of a said sensor and rotationally orienting said sensor about the longitudinal axis of the sensor port and/or grommet, and relative to the jacket.

The sensor orienting notch may be configured to engage with the sensor orienting notch of the grommet and sensor port to allow for the mating of the jacket with the sensor port and grommet in only one relative rotational position.

The jacket may comprise a sensor depth locating feature for engaging with a surface of said sensor and locating the sensor at a desired distance within the first portion of the connector, and/or a desired location on or parallel to the longitudinal axis of the sensor port.

The jacket may comprise two securing members for securing the jacket to either or both of the sensor port and the first portion.

The securing members may be for use in securing the jacket to the first portion.

The securing members may at least partially encircle an outer surface of the first portion from which the sensor port depends.

The securing members may at least partially encircle and are configured to frictionally engage with the outer surface of the first portion such as to secure the jacket to the first portion.

The securing members may comprise locking features to engage with corresponding locking features of the respective first portion.

The locking features of the first portion may be located on substantially opposite sides of said first portion.

The locking features of the respective first portion may be spaced apart from each other along the lumen.

The locking features of the first portion may be aligned substantially parallel to a longitudinal axis of the second portion.

The connector may comprise a cover for coupling with the sensor port and for sealing the sensor port, the cover comprising a projection for location within and a sealing of the sensor port.

The cover may depend from the jacket.

The cover may be connected to the jacket by a tether.

When the cover is not coupled with the sensor port the tether may be configured to be located away from the sensor port.

When the cover is not coupled not coupled with the sensor port the tether may be configured to extend substantially perpendicularly to the longitudinal axis of the sensor port.

The cover may comprise a gripping feature to aid in gripping of the cover by a user

The gripping feature may be an outwardly extending collar.

When the sensor port is sealed by the projection the gripping feature may be presented set away from the jacket such that the gripping feature may be grasped by a user.

The gripping feature may not extend laterally past an extent of a portion of the jacket fitted about the sensor port.

The jacket may comprise an upstand to engage with the gripping feature and provide a limit to the passage of the projection within the sensor port.

The upstand may be comprised by a or the sensor depth locating feature of the jacket.

An external surface of the connector may comprise a plurality of gripping features for grasping by a user.

The gripping features may be configured to present resistance to the fingers of a user during the connection or disconnection of the connector from a gas supply outlet.

The gripping features may comprise at least one pair of ribs or nodes projecting from the external surface of the connector, wherein the ribs or nodes of the at least one pair are presented at substantially opposite external surface portions of the connector.

The gripping features may comprise three pairs of ribs or nodes, and the ribs or nodes of each pair are presented at substantially opposite external surface portions of the connector.

The gripping features may comprise at least one set of ribs, and each rib is oriented such that a longitudinal direction of each rib is substantially non-parallel with a longitudinal axis of the first portion proximate to the first opening.

Each rib may be oriented substantially parallel with a longitudinal axis of the second portion proximate to the second opening.

The connector may comprise a cuff configured to be located over at least part of said second portion.

The connector may comprise a terminal collar, the terminal collar being located away from an end of the second portion (i.e. the second opening) the cuff configured to engage said terminal collar when installed.

The terminal collar may be provided around a circumference of the second portion.

The connector and cuff may comprise corresponding at least one locking feature configured to lock and retain the cuff on the second portion.

The locking feature may comprise a locking collar and a protrusion.

The protrusion may be configured to engage with the locking collar to engage and connect the connector and the cuff.

The locking collar may be provided on an outer surface of the second portion, or the protrusion on an inner surface of the cuff.

The protrusion may be provided on an outer surface of the second portion, or the protrusion on an inner surface of the cuff.

The locking collar may be located around a circumference of an outer surface of the second portion, or an inner surface of the cuff.

The protrusion collar may be located around a circumference of an outer surface of the second portion, or an inner surface of the cuff.

The locking collar may act as a limit surface for an overmould located under the cuff to overmould the interface between the tube and the connector.

The cuff may have a cut out located at one end, the cut out being shaped to accommodate a profile of an electrical port.

The electrical port may be aligned with an outer surface of the connector.

The electrical port may be tangentially aligned with an outer surface of the connector.

The electrical port may extend outwardly from the connector.

The electrical port may extend outwardly from the second portion.

The electrical port may be offset from centre of the connector so as to be aligned with a termination point of the wire from the tube wall.

A termination point of the wire from the tube wall may be aligned with said electrical port.

A direct pathway may be provided from a termination point of the wire from the tube wall to the electrical port.

The electrical port may comprise at least one connection point, the connection point configured to provide for connection of the electrical port to the at least one wire.

Where the connection point may be aligned with an outer surface of the connector

The connection point of the electrical port may be aligned with a surface tangential to an outer surface of the connector.

A termination point of the wire from the tube wall may be aligned with the connection point.

A direct pathway may be provided from a termination point of the wire from the tube wall to the connection point.

The electrical port may comprise at least one pin configured to be in electrical communication with said at least one wire.

The electrical port and/or the connection point of the electrical port may be configured to receive at least one wire of the tube wall, or the pin (for example a crimp pin) connected to said at least one wire.

The at least one wire may comprise a pair of heater wires

The at least one wire may comprise a pair of sensor wires

The electrical port may be formed integrally with the connector.

The electrical port may be in the shape of a cloverleaf.

The electrical port may comprise a first portion, a second portion and a third portion.

The first portion and second portion may comprise the at least one pin configured to be in electrical communication with said at least one wire.

The first portion and second portion may comprise two pins configured to be in electrical communication with said at least one wire.

The third portion may be configured to receive a locating portion of a corresponding electrical port.

The connector may comprise at least one guide feature configured to direct the at least one wire from the tube wall of the tube, and/or a pin connected to said at least one wire, to the electrical port and/or the connection point of the electrical port.

The guide feature may be tangentially arranged on a surface of the connector.

The guide feature may be provided along a plane aligned with the connection point of the electrical port.

Said guide feature may locate said wire and/or pin in a predetermined location.

Wherein at the predetermined location the wire and/or pin may be aligned with the electrical port and/or the connection point of the electrical port.

The at least one guide feature may be or may comprise an outer surface of the connector.

The at least one guide feature may be formed at least in part by one or more recesses or protrusions.

The at least one guide feature may be provided on the second portion of the connector.

The at least one guide feature may comprise at least one rib, wherein the rib is oriented parallel or along a longitudinal axis of the second portion, or a longitudinal axis of the medical tube.

The guide feature may comprise a rib having a recess configured to locate at least one wire and/or said at least one pin within the recess.

The connector may comprise:
a first portion having a first opening configured to receive a flow of gases,
a second portion having a second opening, the second portion configured to engage with the medical tube and provide a flow of gases to the medical tube through the second opening,
a lumen formed by the first portion and the second portion, the lumen providing a gases flow path between the first opening and the second opening.

The tube may further comprise:
a lumen extending from a first end of the tube to a second end of the tube,
a first elongate member comprising a hollow body spirally wound to form at least in part said lumen,
a second elongate member spirally wound and joined between adjacent turns of the first elongate member, and

The first elongate member of the tube may form in longitudinal cross-section a plurality of hollow portions each with a flattened surface forming at least part of the wall surrounding the lumen.

The lumen may be formed by the first elongate member and the second elongate member comprises a substantially smooth bore.

The second elongate member may comprise said at least one wire.

The connector may comprise at least one location feature the location feature configured to locate the second elongate member from the end of the tube into a predetermined location.

The predetermined location may align the second elongate member with the electrical port and/or the connection point of the electrical port.

The predetermined location may allow for the wires from the second elongate member to be provided to corresponding guide features.

The location features may comprise one or more ribs, and/or recesses, oriented parallel or along a longitudinal axis of the second portion, or a longitudinal axis of the medical tube.

The location features may comprise a rib having a recess, the recess configured to locate said second elongate member to locate said second elongate member at said predetermined location.

The second portion may comprise at least one connection feature

The connection feature may be configured to engage and retain the medical tube.

The connection feature may be a thread.

The guide surface may be located at the termination of the connection feature, so as to be provided at the end of the medical tube when the medical tube is attached to the connector.

The connector may comprise at least one protrusion located at or near the end of the thread, the protrusion configured to provide a stop to engage with the medical tube and prevent further engagement of the medical tube and the thread.

The connection between the medical tube and the connector may be overmoulded.

In another aspect there is provided a connector for a medical tube, wherein the connector comprises:
a first portion having a first opening configured to receive a flow of gases,
a second portion having a second opening, the second portion configured to engage with a medical tube and provide a flow of gases to the medical tube through the second opening,
a lumen formed by the first portion and the second portion, the lumen providing a gases flow path between the first opening and the second opening,
wherein the first portion of the connector comprises a sensor port, the sensor port configured to receive a sensor, wherein the sensor port is located on a side of the first portion of the connector,
wherein the second portion of the connector comprises at least one electrical port, the electrical port configured to provide an electrical connection to at least one wire in the tube, and wherein the electrical port is located on the top of the second portion of the connector.

In another aspect there is provided a connector for a medical tube, wherein the connector comprises:
a first portion having a first opening configured to receive a flow of gases,
a second portion having a second opening, the second portion configured to engage with a medical tube and provide a flow of gases to the medical tube through the second opening,
a lumen formed by the first portion and the second portion, the lumen providing a gases flow path between the first opening and the second opening,
wherein the first portion of the connector comprises a sensor port, the sensor port configured to receive a sensor,
wherein the second portion of the connector comprises at least one electrical port, the electrical port configured to provide an electrical connection to at least one wire in the tube,
and wherein the sensor port is offset about 120 to about 75 degrees, or about 110 to about 80 degrees, or about 90 degrees, about a longitudinal axis of the lumen relative to the electrical port.

The first portion may be angled with respect to the second portion to form an elbow

The angle between the first portion and the second portion may be about 90 degrees to about 170 degrees, or about 100 degrees to about 150 degrees, or about 110 to about 140 degrees, or about 135 degrees.

The first portion may provide for a taper fit connection.

The sensor port may provide an aperture for the sensor to be inserted so as to be in contact with a flow of gases in the first portion of the connector.

The sensor may be configured to measure flow rate of the flow of gases in the first portion of the connector and/or measure temperature of the flow of gases in the first portion of the connector. and/or measure humidity of the flow of gases in the first portion of the connector.

The sensor may be comprised as part of a probe, the probe for being received by the sensor port.

The sensor port is offset may be about 120 to about 75 degrees, or about 110 to about 80 degrees, or about 90 degrees, about a longitudinal axis of the lumen relative to electrical port.

Opposite sides of the elbow may define internal and external elbow surfaces, and wherein the electrical port may be located on the external elbow surface.

The sensor port may be oriented perpendicular to the first portion.

the sensor port may be oriented perpendicular to a longitudinal axis of the first portion.

The electrical port may be oriented perpendicular to the second portion.

The electrical port may be located tangential to an outer surface of the connector.

The electrical port may be offset from a longitudinal central axis of the second portion of the connector.

The sensor port may have a longitudinal axis, the sensor port allowing communication with an interior of the connector

The longitudinal axis of the sensor port may be oriented substantially perpendicularly to the flow of gases through the first portion of the connector.

The longitudinal axis of the sensor port may substantially intersects a corresponding longitudinal axis of the first portion of the connector from which the sensor port depends.

The sensor port may comprise a substantially cylindrical cross-section.

A terminal surface of the sensor port distal of an outer surface of the first portion may comprise one or more locating features.

The one or more locating features may comprise one or more notches of the terminal surface of the sensor port.

The one or more notches may extend in the direction of the longitudinal axis of the sensor port.

The connector may comprise a grommet, the grommet configured to be located with the sensor port.

The grommet may be integrally formed with the sensor port, and/or a jacket.

The locating features may comprise a plurality of notches, the notches spaced apart about a perimeter of the terminal surface and at least one of the plurality of notches configured to mate with a corresponding number of projections of the grommet.

The grommet may comprises a sensor orienting notch for receiving a projection of a or the sensor and rotationally orienting said sensor about the longitudinal axis of the sensor port.

The sensor port may comprise a sensor orienting notch for receiving a projection of a or the sensor and rotationally orienting said sensor about the longitudinal axis of the sensor port.

The or a grommet may comprise a sensor locating feature for engaging with a surface of a sensor and locating said sensor at a desired location within the sensor port.

The sensor locating feature may comprise a radially extending lip spaced away from the terminal surface of the sensor port and projecting inwardly towards the longitudinal axis of the sensor port.

The connector may comprises a jacket for the sensor port, the jacket for fitment at least partially about the sensor port, and/or the grommet.

The jacket may comprise a sensor orienting notch for receiving a projection of a said sensor and rotationally orienting said sensor about the longitudinal axis of the sensor port and/or grommet, and relative to the jacket.

The sensor orienting notch may be configured to engage with the sensor orienting notch of the grommet and sensor port to allow for the mating of the jacket with the sensor port and grommet in only one relative rotational position.

The jacket may comprise a sensor depth locating feature for engaging with a surface of said sensor and locating the sensor at a desired distance within the first portion of the connector, and/or a desired location on or parallel to the longitudinal axis of the sensor port.

The jacket may comprise two securing members for securing the jacket to either or both of the sensor port and the first portion.

The securing members may be for use in securing the jacket to the first portion.

The securing members may at least partially encircle an outer surface of the first portion from which the sensor port depends.

The securing members may at least partially encircle and may be configured to frictionally engage with the outer surface of the first portion such as to secure the jacket to the first portion.

The securing members may comprise locking features to engage with corresponding locking features of the respective first portion.

The locking features of the first portion may be located on substantially opposite sides of said first portion.

The locking features of the respective first portion may be spaced apart from each other along the lumen.

The locking features of the first portion may be aligned substantially parallel to a longitudinal axis of the second portion.

The connector may comprise a cover for coupling with the sensor port and for sealing the sensor port, the cover comprising a projection for location within and a sealing of the sensor port.

The cover may depends from the jacket.

The cover may be connected to the jacket by a tether.

When the cover is not coupled with the sensor port the tether may be configured to be located away from the sensor port.

When the cover is not coupled with the sensor port the tether may be configured to extend substantially perpendicularly to the longitudinal axis of the sensor port.

The cover may comprise a gripping feature to aid in gripping of the cover by a user

The gripping feature may be an outwardly extending collar.

The when the sensor port may be sealed by the projection the gripping feature is presented set away from the jacket such that the gripping feature may be grasped by a user.

The gripping feature may not extend laterally past an extent of a portion of the jacket fitted about the sensor port.

The jacket may comprise an upstand to engage with the gripping feature and provide a limit to the passage of the projection within the sensor port.

The upstand maybe comprised by a or the sensor depth locating feature of the jacket.

The electrical port may be configured to extend in a direction upward and away from the humidifier when the connector is connected to a humidification chamber.

The electrical port may be configured extend in a direction away from the humidifier when the connector is connected to a humidification chamber.

In another aspect there is provided a medical tube assembly, the medical tube assembly comprising:
a tube, the tube comprising:
   a lumen extending from a first end of the tube to a second end of the tube,
   a first elongate member comprising a hollow body spirally wound to form at least in part said lumen,
   a second elongate member spirally wound and joined between adjacent turns of the first elongate member, and
   wherein a first end of the tube comprises at least one connector,
the connector comprising:
   a first portion having a first opening configured to receive a flow of gases,
   a second portion having a second opening, the second portion configured to engage with a medical tube and provide a flow of gases to the medical tube through the second opening,
   a lumen formed by the first portion and the second portion, the lumen providing a gases flow path between the first opening and the second opening,
   wherein the first portion is angled with respect to the second portion to form an elbow.

The first portion of the connector may comprise a sensor port, the sensor port configured to receive a sensor.

The second portion of the connector may comprise at least one electrical port, the electrical port configured to provide an electrical connection to the or at least one wire in the tube.

In another aspect there is provided a medical tube assembly, the medical tube assembly comprising:
a tube, the tube comprising:
   a lumen extending from a first end of the tube to a second end of the tube,
   a first elongate member comprising a hollow body spirally wound to form at least in part said lumen,
   a second elongate member spirally wound and joined between adjacent turns of the first elongate member, and
   wherein a first end of the tube comprises at least one connector,
the connector comprising:
   a first portion having a first opening configured to receive a flow of gases,
   a second portion having a second opening, the second portion configured to engage with a medical tube and provide a flow of gases to the medical tube through the second opening,
   a lumen formed by the first portion and the second portion, the lumen providing a gases flow path between the first opening and the second opening
   the first portion of the connector comprises a sensor port, the sensor port configured to receive a sensor.

The second portion of the connector may comprises at least one electrical port, the electrical port configured to provide an electrical connection to the or at least one wire in the tube.

In another aspect there is provided a medical tube assembly, the medical tube assembly comprising:
a tube, the tube comprising:
   a lumen extending from a first end of the tube to a second end of the tube,
   a first elongate member comprising a hollow body spirally wound to form at least in part said lumen,
   a second elongate member spirally wound and joined between adjacent turns of the first elongate member, and
   wherein a first end of the tube comprises at least one connector,
the connector comprising:
   a first portion having a first opening configured to receive a flow of gases,
   a second portion having a second opening, the second portion configured to engage with a medical tube and provide a flow of gases to the medical tube through the second opening,
   a lumen formed by the first portion and the second portion, the lumen providing a gases flow path between the first opening and the second opening
   wherein the second portion of the connector comprises at least one electrical port, the electrical port configured to provide an electrical connection to the or at least one wire in the tube.

The electrical port may be located tangential to an outer surface of the connector.

In another aspect there is provided a medical tube assembly, the medical tube assembly comprising:
a connector, the connector being the connector of as described above.
a tube, the tube comprising:
   a lumen extending from a first end of the tube to a second end of the tube,
   a first elongate member comprising a hollow body spirally wound to form at least in part said lumen,
   a second elongate member spirally wound and joined between adjacent turns of the first elongate member.

In another aspect there is provided a medical tube assembly, the medical tube assembly comprising:
a connector, the connector being the connector as described above.
a tube, the tube comprising:
   a lumen extending from a first end of the tube to a second end of the tube,
   a first elongate member comprising a hollow body spirally wound to form at least in part said lumen,
   a second elongate member spirally wound and joined between adjacent turns of the first elongate member.

The second elongate member may form at least a portion of the lumen.

The first elongate member of the tube may form in longitudinal cross-section a plurality of hollow portions each with a flattened surface forming at least part of the wall surrounding the lumen

The medical tube may comprises at least one wire.

The wire may be located between the plurality of hollow portions and the lumen of the tube.

The wire may be located within the second elongate member.

The lumen may beformed by the first elongate member and the second elongate member comprises a substantially smooth bore.

An outer surface of the tube is corrugated, and optionally the corrugations are annular or helical.

The at least one wire may extend along a portion of the length of the medical tube.

The at least one wire may comprises at least one heater wire.

The at least one wire comprises a pair of heater wires.

The at least one wire comprises at least one sensor wire.

The at least one wire may comprise a pair of sensor wires.

The heating wire may be located within the lumen of the medical tube.

The heating wire may be attached to an inner wall of the medical tube.

The heating wire may be embedded within a wall of said medical tube.

The wire is embedded within a second elongate member.

An internal diameter of the lumen of the medical tube may be about 10 mm to about 25 mm, or about 13 mm to about 20 mm, or about 16 mm to about 18 mm, or about 17.

A length of the medical tube may be about 1.4 m to about 1.8 m or about 1.5 m to about 1.7 m.

The medical tube may be a respiratory tube or an inspiratory tube.

The connector may comprise the features of any one or combination of the other aspects.

In another aspect there is provided a medical tube assembly, comprising a connector, the connector being the connector of any one or combination of the other aspects, and a tube, wherein a lumen of the tube comprises a substantially smooth bore.

When the tube is provided to the second portion of the connector and engaged with said connection features, the wires of the tube extending from the tube wall may be aligned with said electrical port.

The connector may comprise:
a first end having a first opening, and
a second end having a second opening,
the connector configured to engage with the medical tube at the second end and receive a flow of gases from the medical tube through the second opening,
a lumen formed by the connector, the lumen providing a gases flow path between the second opening and the first opening.

The first end of the connector may be configured for engagement with a patient interface, and/or a patient interface extension tube.

The connector may comprise at least one sensor port.

The sensor port may extend outwardly from the connector.

The sensor port may comprise at least one cover.

The cover may be attached to the connector about the sensor port.

The cover may be retained around the sensor port by one or more retention features.

The retention features may comprise one or more projections.

The cover may comprise a cap connected to the cover by a tether.

The connector may comprise at least one connection feature configured to allow for connection with the medical tube.

The connection feature may be a thread.

The connector may comprise at least one protrusion located at or near the end of the thread, the protrusion configured to provide a stop to engage with the medical tube and prevent further engagement of the medical tube and the thread.

The connector may comprise a cuff configured to be located over at least part of said second portion.

The connector may comprise a terminal collar, the first collar being located away from the second end the cuff configured to engage said terminal collar when installed.

The terminal collar may be provided around a circumference of the second portion.

The connector and cuff may comprise corresponding at least one locking feature configured to lock and retain the cuff on the second portion.

The locking feature may comprise a locking collar and a protrusion.

The protrusion may be configured to engage with the locking collar to engage and connect the connector and the cuff.

The locking collar may be provided on an outer surface of the second portion, or the protrusion on an inner surface of the cuff.

The protrusion may be provided on an outer surface of the second portion, or the protrusion on an inner surface of the cuff.

The locking collar may be located around a circumference of an outer surface of the second portion, or an inner surface of the cuff.

The protrusion collar may be located around a circumference of an outer surface of the second portion, or an inner surface of the cuff.

The locking collar may act as a limit surface for an overmould located under the cuff to overmould the interface between the tube and the connector.

The cuff and the connector may comprise corresponding alignment features.

The alignment features may comprise at least one protrusion and a corresponding at least one recess.

The alignment features may be configured to prevent relative rotation between the cuff and the connector.

The at least one protrusion may comprise one or more teeth.

The cuff may comprise said at least one recess, and the connector comprises said at least one protrusion.

The connector may comprise said at least one recess, and the cuff comprises said at least one protrusion.

The at least one recess may be located on an internal surface of the cuff.

The at least one recess may comprise a plurality of recesses, such that the cuff may engage with the protrusions in a plurality of orientations.

The at least one recess may be located around the entire circumference of the internal surface of the cuff.

The at least one protrusion may be located on the connector.

The at least one protrusion may be located on a or the terminal collar or a or the locking collar of connector.

The connector may comprise a wire termination feature, the wire termination feature configured to provide for termination of a or the wire of the tube at the connector.

The wire termination feature may comprise one or more projections extending in a direction away from the connector.

In another aspect there is provided a medical tube as described with respect to any of the above aspects, wherein the medical tube comprises a further connector located at a second end of the medical tube, the further connector defined by the connector of above features.

In another aspect there is provided a breathing circuit kit comprising:
the connector, or medical tube, or medical tube assembly of any of any preceding clauses; and
at least one of: a dry line, a filter, an expiratory port, a humidification chamber, a pressure line, an interface extension tube.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7).

Embodiments described herein can also be said broadly to relate to the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

In this specification, where reference has been made to external sources of information, including patent specifications and other documents, this is generally for the purpose of providing a context for discussing the features of the present technology.

Unless stated otherwise, reference to such sources of information is not to be construed, in any jurisdiction, as an admission that such sources of information are prior art or form part of the common general knowledge in the art.

The term "comprising" as used in this specification means "consisting at least in part of". When interpreting statements in this specification which include that term, the features, prefaced by that term in each statement, all need to be present but other features can also be present. Related terms such as "comprise" and "comprised" are to be interpreted in the same manner.

### BRIEF DESCRIPTION OF THE DRAWINGS

The invention will now be described by way of example only and with reference to the drawings in which:
Figures 1A and 1B show embodiments of respiratory system.
Figures 2A to 2D show embodiments of a medical tube.
Figure 2E shows a cross-sectional view of a portion of a medical tube.
Figures 3 to 3B show embodiments of a medical tube
Figure 4 shows an embodiment of a medical tube assembly having a medical tube and one or more connectors.
Figures 5A and 5B shows an embodiment of a connector as part of a medical tube assembly.
Figure 6 shows a connector as part of a medical tube assembly.
Figure 7 shows a connector of a medical tube assembly.
Figure 7A shows a grommet and jacket of a sensor port.
Figure 8 shows a connector of a medical tube assembly.
Figure 8A shows a sensor probe.
Figure 8B shows a cuff of a connector.
Figures 9 and 10 show a connector of a medical tube assembly.
Figures 11 to 13 show another connector of a medical tube assembly.

### DETAILED DESCRIPTION

Reference is made in detail to an embodiment of the present technology, examples of which is illustrated in the accompanying drawings.

Medical tubes can be used in breathing circuits or respiratory systems, for example, for delivering and/or removing humidified gases from a patient, such as in obstructive sleep apnea, neonatal, respiratory humidification, and surgical humidification systems including insufflation systems and systems for patients undergoing procedures under general anesthetic.

This application relates to medical tube assemblies, medical tubes and connectors for use in breathing circuits or respiratory systems. The medical tubes and connectors may be used for delivering and/or removing humidified gases from a patient, such as in obstructive sleep apnea, neonatal, respiratory humidification, and surgical humidification systems including insufflation systems and systems for patients undergoing procedures under general anesthetic.

The medical tube assemblies can be used to deliver gases between two components of a breathing circuit. Medical tube assemblies can be used to deliver gases between a component and a patient. For example between a humidifier and a patient.

In some embodiments, the medical tubes can be inspiratory tubes, expiratory tubes, patient interface tubes, supply tubes, dry lines, insufflation tubes, etc.

The medical tube assemblies may comprise one or more medical tubes, and one or more connectors.

Connectors may allow for properties of the gases to be sensed without compromising the integrity of the tube, or allowing leakage of gases from or to the external environment.

A sensor port may be provided to allow for the insertion of a sensor through the connector wall and into the gases flow path.

In some systems the connector can also provide an electrical interface (for example an electrical port) between a respiratory device and wires provided in the tubes. The wires may be for example heater wires and/or sensor wires.

In cases where the sensor and electrical ports are required cable management can be difficult as there may be separate wires going to each of the sensor and the electrical port. Removal and insertion of probes and/or electrical ports may become difficult given the small size of the connector and the various cabling required.

As described below in more detail the sensor port and heater wire may be provided at different portions of a connector, and extend in different directions so as to not interfere with each other.

Tubes for use in respiratory device may be designed to minimise condensation (for example by including insulation and/or heater wires) for example see: PCT Publication No. WO2012164407.

Tubes for use in respiratory device may be designed to minimise resistance to flow for example see: PCT Publication No. WO2018016975, PCT Publication No. WO2011/149362, and PCT Application No. PCT/NZ2019/050016.

As described in more detail below, resistance to flow may be an important consideration in designing a tubing assembly. Resistance to flow may be minimised by increasing the diameter of the tubing to increase the cross-sectional area of the gases flow path. As a consequence of increasing the diameter of the tubing an increased heating of the gases by a heating element may be required to maintain the humidified gases above their dew point to prevent the formation of condensate. Given a tube of increased diameter it may not be possible to heat the gases to the required dewpoint.

Smooth bore tubes may be provided to decrease the resistance to flow of gases. Furthermore, a decrease in resistance to flow of gases provided by a smooth bore tube may allow for a decrease in the internal diameter of the tubing. However, challenges such as connection with an internal smooth bore, and with the wires (for example heater wires of the tube) may be associated with providing connectors to smooth bore tubing.

The medical tubes, connectors and medical tube assemblies described herein can be provided in one or more respiratory systems, breathing circuits, or kits.

The medical tubes may be used for delivering and/or removing humidified gases from a patient, such as in obstructive sleep apnea, neonatal, respiratory humidification, and surgical humidification systems including insufflation systems and systems for patients undergoing procedures under general anesthetic. The medical tubes can be used to deliver respiratory gases to and/or from a patient as part of a respiratory therapy or treatment. The respiratory gases may be heated and/or humidified prior to delivery to the patient in order to, for example, reduce the likelihood of infection and/or tissue damage.

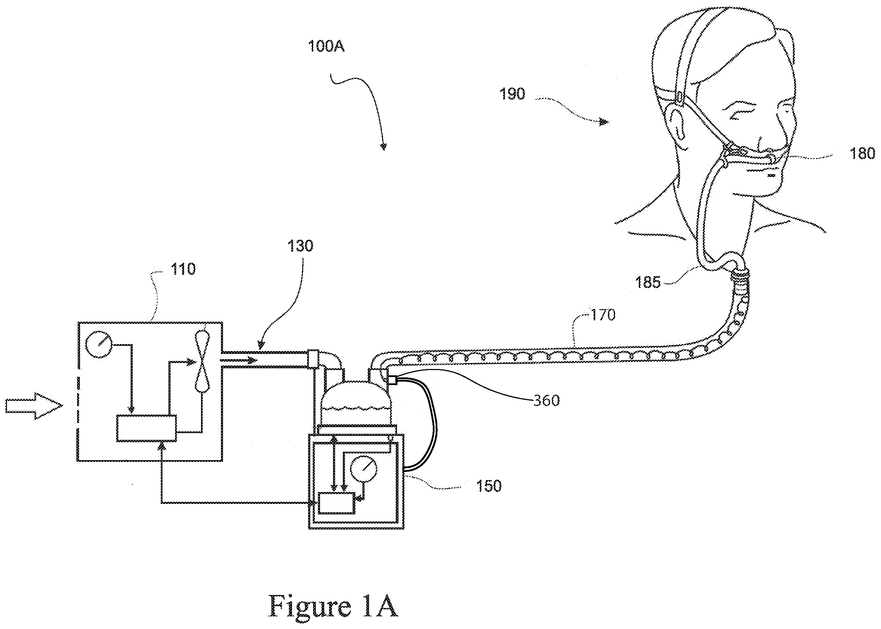

FIG. 1A schematically illustrates an embodiment of respiratory system (or breathing circuit) 100A that can include one or more of the medical tubes described herein. In the illustrated embodiment, the respiratory system 100A includes a gases source 110 that is either integrated with, or a separate component from, a humidification apparatus 150 (e.g., a humidifier).

The gases source 110 and/or humidification apparatus 150 supply heated and humidified gases to a patient 190 via a breathing circuit that includes, for example, an inspiratory tube 170 and a patient interface 180.

As used herein, patient interface has a broad meaning and is to be given its ordinary and customary meaning to one of skill in the art, and patient interface also includes, without any limitation, any one or more of a full face mask, a nasal mask, an oral mask, an oral-nasal mask, a nasal pillows mask, nasal cannulas, nasal prongs, a laryngeal mask, or any other suitable coupling between the medical circuit and the airways of the patient.

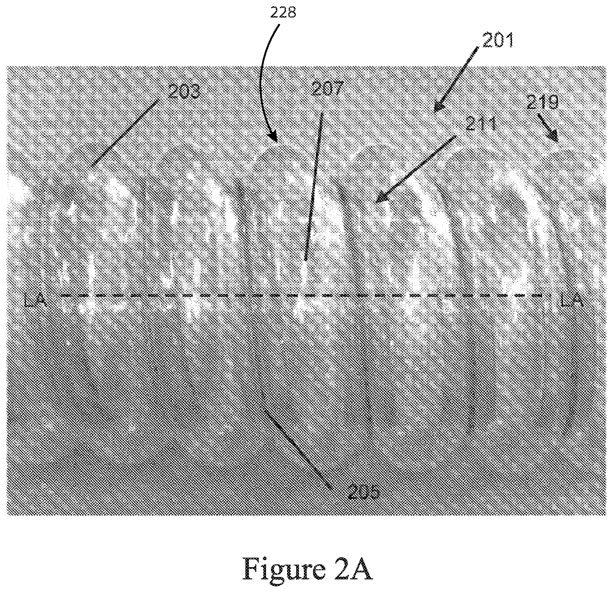

In some embodiments, the inspiratory tube 170 can be any of the medical tubes described herein (for example, the inspiratory tube 170 can be any of the medical tubes 201, 220, 222, 225 shown in FIGS. 2A-3B and described below).

In some embodiments, another medical tube, such as a supply tube 130, can be used to transport gases from the gases source 110 to the humidification apparatus 150. Supply tube 130 is sometimes called a "dry" line, as it is positioned in the breathing circuit prior to the "wet" humidifier. In some embodiments, the supply tube/dry line 130 can be any of the medical tubes described herein (for example, the tube/dry line 130 can be any of the medical tubes 201, 220, 222, 225 shown in FIGS. 2A-3B and described below).

In some embodiments, an additional tube, such as an interface tube 185, can connect between the inspiratory tube 170 and the patient interface 180. It is to be understood that other variations from the system 100A shown may exist. For example, the inspiratory tube 170 may comprise multiple sections to accommodate other equipment such as a water trap, an intermediate connector with one or more sensors, a PCB, and/or a controller.

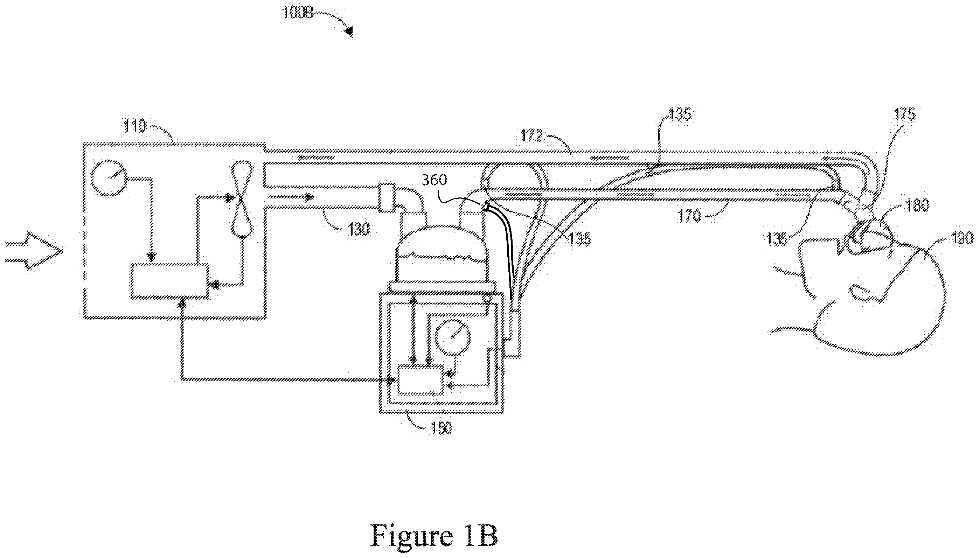

Figure 1B schematically illustrates another embodiment of respiratory system (or breathing circuit) 100B that can include one or more of the medical tubes described herein. In many respects, the respiratory system 100B can be similar to the respiratory system 100A of FIG. 1. For example, as illustrated, the respiratory system 100B includes a gases source 110 and a humidification apparatus 150 (e.g., a humidifier). The gases source 110 and/or humidification apparatus 150 supplies heated and humidified gases to a patient 190 via a breathing circuit that includes, for example, an inspiratory tube 170 and a patient interface 180. In FIG. 1B, however, the breathing circuit further includes an expiratory tube 172, by which exhaled gases can be transported. In some embodiments, the expiratory tube 172 transports exhaled gases back to the gases source 110 and/or humidification apparatus 150.

In the illustrated embodiment of FIG. 1B, the connector 175 can comprise a y (wye) piece that connects both the inspiratory tube 170 and the expiratory tube 172 to a patient interface component, such as the interface tube 185 (as shown in Fig. 1A), or directly to the patient interface 180 itself. Further, the respiratory system 100B can include one or more sensors 135.

For example, a sensor 135 can connect to the inspiratory tube 170 near the patient interface 180 or a sensor 135 can connect to the patient interface 180, among other possible sensor locations. The sensor 135 can be integrated into or connectable to the inspiratory tube 170. In the illustrated embodiment, the system 100B includes two sensors 135, with a first sensor 135 positioned at or nearby to the humidifier chamber outlet end of the inspiratory tube 170, and a second sensor 135 positioned at the patient end of the inspiratory tube 170.

In some embodiments, the inspiratory tube 170 can comprise the medical tube 201, 220, 222, 225 shown in FIGS. 2A-3B and described below and the sensors 135 can be connected to the sensor port 340 of the connector 300 and/or the sensor port 605 of the connector 600. Alternatively or in addition, sensor(s) 135 may be provided at or nearby to the humidifier chamber inlet. A signal provided by the sensor(s) 135 can be provided, for example, to a control system. In some embodiments, the sensor(s) 135 comprises one or more of a temperature sensor, a humidity sensor, a flow sensor, and a pressure sensor. Although the sensors 135 are illustrated connected to the patient end and chamber outlet end of the inspiratory tube 170, one or more sensors can be included, alternatively or additionally, in other locations on the inspiratory tube 170 and/or on other medical tubes or components in the respiratory system.

It is to be understood that other variations from the system shown may exist. For example, the inspiratory 170 and/or expiratory tube 172 may comprise multiple sections to accommodate other equipment such as a water trap, an intermediate connector with one or more sensors, a PCB, and/or a controller. In another example embodiment, the system may include a nebulizer or a port therefore. In another example embodiment, the system may include a catheter mount or an exhalation valve.

The medical tube assemblies, medical tubes and/or connectors may also be provided as a breathing kit. The breathing kit may also comprise at least one of: a dry line 130, a filter, an expiratory port, a humidification chamber, a pressure line, an interface extension tube.

A tube may have a lumen extending from a first end of the tube to a second end of the tube.

The tube may form part of a tube assembly, the tube assembly may comprise at connector at a first end of the tube. In some embodiments, the tube may comprise a further or second connector at a second end of the tube.

As described in more detail below the tube may comprise one or more wires.

The wires may be a heater wire, and/or a sensor wire.

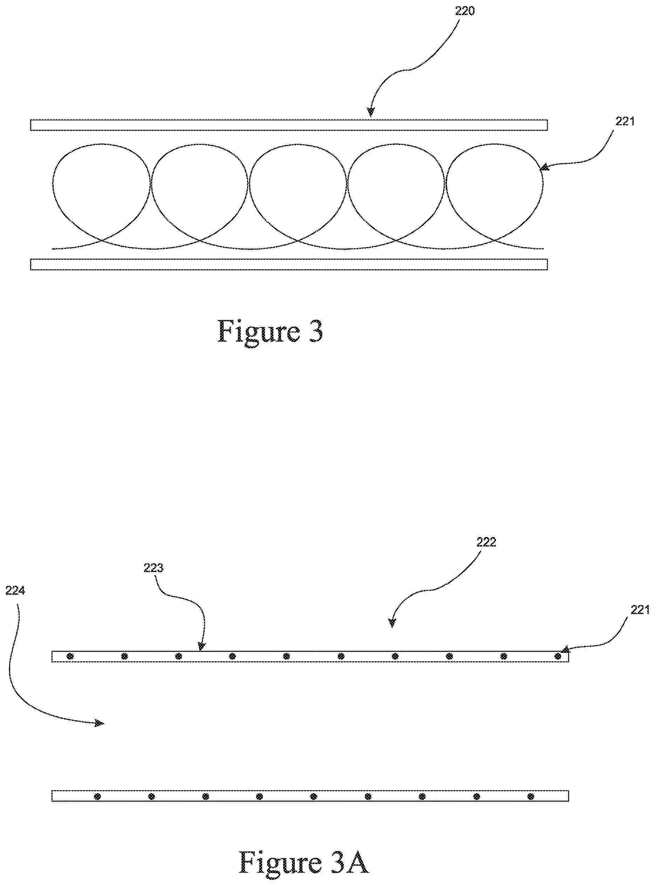

The wires may be located in the wall of the tube (For example as shown in Figure 2A to 2C or Figure 3A)

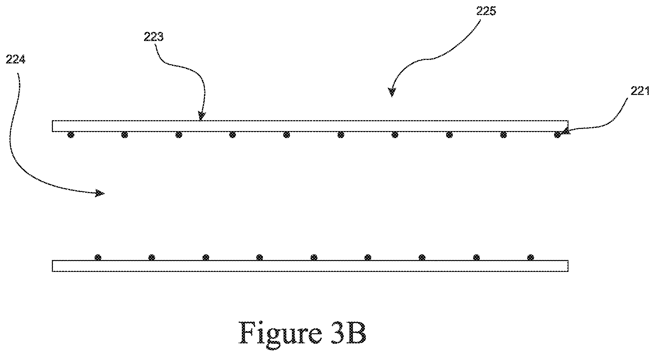

The wires may be located in the wall or the located within a lumen of the tube (For example as shown in Figure 3).

The wires may comprise may comprise at least one heater wire.

The wires may comprise a pair of heater wires.

The wires may comprise at least one sensor wire.

The wires may comprise a pair of sensor wires.

The wires may be located along a portion of the length of the tube, or along the entire length of the tube.

In some embodiments the tube may be spirally wound. Spirally wound tubes may for example comprise one or more member(s) which is wound spiral to form a lumen.

In some embodiments the tube may be annularly wound. Annular tubes may comprise a series of members wound annularly to form a lumen.

The tube may comprise an externally corrugated surface for example as shown in Figure 2A-2C.

Described below are examples of different types of tubes.

The tube may be part of a medical tube assembly along with at least a connector.

Figure 2A shows a side plan view of a section of a medical tube 201. The medical tube 201 may be a respiratory tube for example an inspiratory tube. In general, the tube 201 comprises a first elongate member 203 and a second elongate member 205. Member is a broad term and is to be given its ordinary and customary meaning to a person of ordinary skill in the art (i.e., it is not to be limited to a special or customized meaning) and includes, without limitation, integral portions, integral components, and distinct components. The first elongate member 203 may have a hollow portion 228, while the second elongate member 205 may be a structural support or reinforcement which adds structural support to the hollow body.

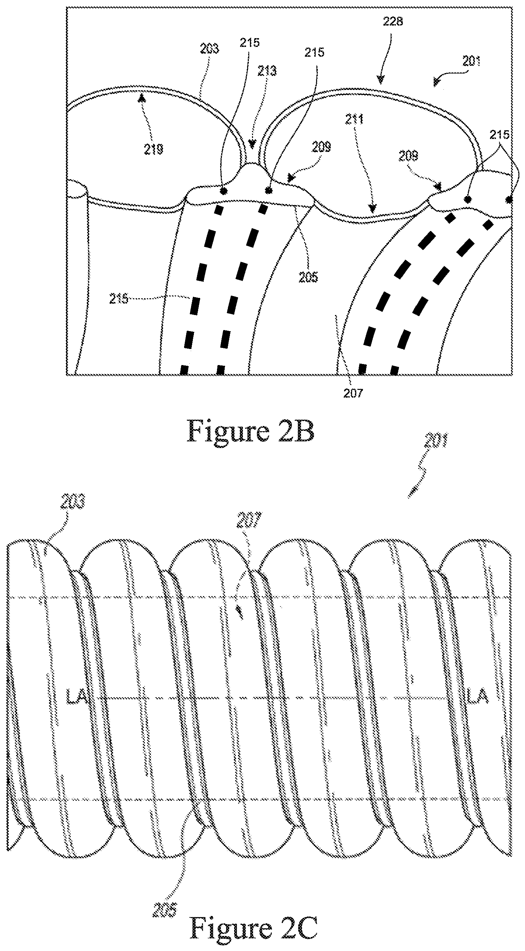

Figure 2B shows a longitudinal cross-section of a top portion of the tube 201 of Figure 2A. Figure 2B has the same orientation as Figure 2A. The first elongate member 203 can have a hollow-body shape. The first elongate member 203 can form in longitudinal cross-section a plurality of hollow portions 228. Portions 209 of the first elongate member 203 can overlap adjacent wraps of the second elongate member 205. A portion 211 of the first elongate member 203 can form at least part of the wall of the lumen 207 (tube bore). Adjacent hollow portions can be separated by a gap 213. A T-shaped second elongate member 205, as shown in Figure 2B, can help maintain a gap 213 between adjacent hollow portions 228.

As described in more detail below, figure 2B shows wires 215 visible in second elongate member however it will be appreciated these wires 215 may not be visible is the second elongate member is opaque.

Figure 2C shows a side view of a portion of the tube 201, with the first elongate member 203 and the second elongate member 205 forming lumen 207.

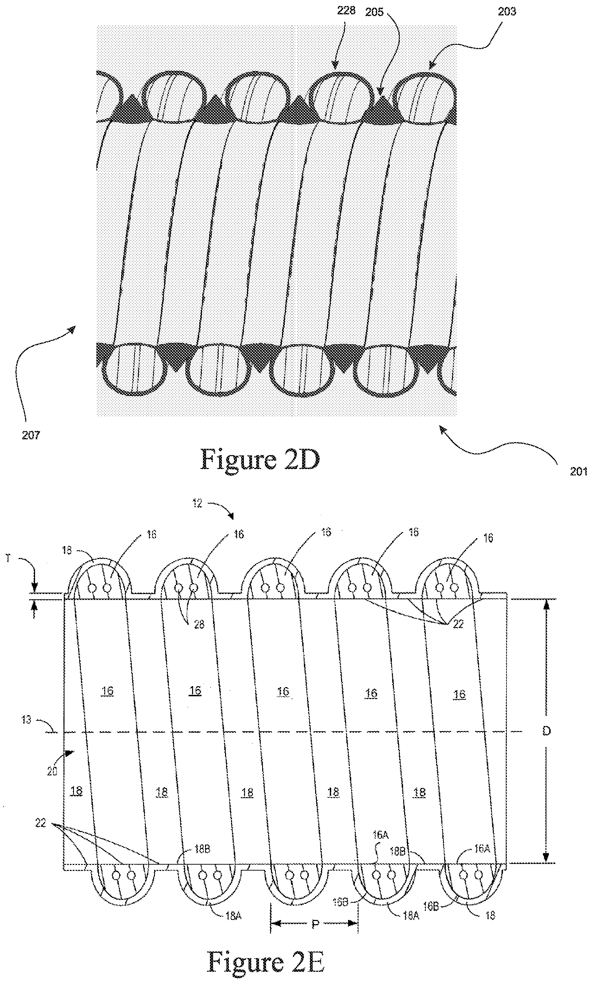

In some embodiments, the lumen 207 may have a smooth bore (described in more detail below.) Figure 2D shows an example of such a tube 201 having an internal smooth bore.

The first elongate member 203 can comprise a hollow body spirally wound to form, at least in part, an elongate tube having a longitudinal axis LA- LA and a lumen 207 extending along the longitudinal axis of the tube, when the tube is in an extended configuration LA-LA. The first elongate member 203 can form in longitudinal cross-section a plurality of hollow portions. A portion 211 of the first elongate member 203 forms at least part of the inner wall of the lumen 207. The first elongate member 203 can be a tube. In some embodiments, the first elongate member 203 is flexible. Flexible refers to the ability to bend to endure strain without being permanently modified or broken.

Furthermore, the first elongate member 203 may be transparent or, at least, semi-transparent or semi-opaque. A degree of optical transparency allows a caregiver or user to inspect the lumen 207 for blockage or contaminants or to confirm the presence of condensation. A variety of plastics, including medical grade plastics, are suitable for the body of the first elongate member 203. Suitable materials include Polyolefin elastomers, Polyether block amides, Thermoplastic co-polyester elastomers, EPDM-Polypropylene mixtures, and Thermoplastic polyurethanes.

In some embodiments, the first elongate member 203 may be opaque.

The hollow body structure of the first elongate member 203 can contribute to the insulating properties of the inspiratory tube 201. An insulating conduit can help prevent heat loss. This can allow the inspiratory tube 201 to deliver gases from the humidifier 107 to the patient 101 while maintaining the conditioned state of the gases with minimal energy consumption.

The second elongate member 205 can also be spirally wound. The second elongated member 205 can be joined to the first elongate member 203 between adjacent turns of the first elongate member 203. The second elongate member 205 can form at least a portion of the lumen 207 of the elongate tube. The second elongate member 205 can act as structural support for the first elongate member 203. The second elongate member 205 can be wider at the base (proximal the lumen 207) and narrower at the top.

The second elongate member can be generally triangular in shape, generally T-shaped, or generally Y-shaped, or substantially triangular. However, any shape that meets the contours of the corresponding first elongate member 203 is suitable.

The second elongate member 205 can be flexible, to facilitate bending of the tube. In exemplary embodiments, the second elongate member 205 is less flexible than the first elongate member 203. This can improve the ability of the second elongate member 205 to structurally support the first elongate member 203. The second elongate member 205 can be solid or mostly solid.

A variety of polymers and plastics, including medical grade plastics, are suitable for the body of the second elongate member 205. Suitable materials include Polyolefin elastomers, Polyether block amides, Thermoplastic co-polyester elastomers, EPDM-Polypropylene mixtures and Thermoplastic polyurethanes. The first elongate member 203 and the second elongate member 205 may be made from the same material.

The second elongate member 205 can encapsulate or house one or more wires 215. For example the wires 215 may be located within the second elongate member 205.

In exemplary embodiments, the second elongated member 205 may house wires 215 used to transmit information from one or more sensors (not shown). Sensors can include any device configured to detect one or more conditions in the system. For example, sensors can include thermocouples or like thermal sensors, flow sensor, humidity sensor, barometer sensor, and the like.

In some embodiments, the wire 215 may be a heater wire to act as a heating element. A wire 215 can be a resistive heater. In exemplary embodiments, the second elongated member 205 can house the wire of one or more heating elements. The wire 215 can be used to generate heat through electrical resistance. Heating elements can reduce the cold surfaces onto which condensate from moisture-laden gases can form. Heating elements can also be used to alter the temperature profile of gases in the lumen 207 of the inspiratory tube 201.

In exemplary embodiments, two heating wires (for example two wires 215) can be encapsulated in the second elongate member 205, one on either side of the vertical portion of the "T." In some embodiments the second elongate member 205 is, or generally Y-shaped, or substantially triangular. The heating wire (for example wire 215) can include conductive material, such as alloys of Aluminium (Al) and/or Copper (Cu), or conductive polymer. In some embodiments, the material forming the second elongate member 205 is selected to be non-reactive with the metal in the heating wire when the heating wires reach their operating temperature. The material forming the second elongated member 205 can also desirably include a material whose melting temperature is sufficiently high so that elongated member 205 can maintain it structural properties when heating wires reach their operating temperature.

The heater wire 215 may be spaced away from the lumen 207 so that the elements are not exposed to the lumen 207. At one end of the composite tube, pairs of elements can be formed into a connecting loop. A plurality of wires can be disposed in the second elongate member 205.

In some embodiments, the wire 215 may comprise at least one heater wire, and optionally pair of heater wires.

In some embodiments, the wire 215 may comprise at least one sensor wire, and optionally a pair of sensor wires.

FIG. 2E is a cross-sectional view of a portion of the medical tube. As illustrated, the tube body 12 of the medical tube can be formed from a bead 16 and tape or film 18. As will be described in greater detail, the bead 16 and the film 18 can define a bore (or inner lumen) 20 of the medical tube.

An inner surface 22 of the medical tube 10 may at least partially define the lumen (for example bore 20). The inner surface 22 can be substantially smooth. Thus, the lumen bore 20 can be considered (and is often referred to herein as) a substantially smooth bore. As will be described in greater detail below, the inner surface 22 can comprise alternating portions of the bead 16 and the tape or film 18. For example, as illustrated in FIG. 2E, the inner surface 22 can comprise portions 16A of the bead 16 and portions 18B of the film 18. The portions 16A of the bead 16 and the portions 18B of the film 18 can be substantially flat as described below.

It should be appreciated by one of skill in the art that the bead, which may be much thicker and/or made of a harder or more rigid material than the film may impart structural support, reinforcement, and/or resistance to crushing to the more flexible film portions of the tube. Such structural support, reinforcement, and/or resistance to crushing may be important in medical tubes, and in particular for breathing tubes, which must meet international standards defining usage characteristics and parameters.

Forming the inner surface 22 and the bore 20 in this manner (with alternating portions of the bead 16 and the film 18) can be achieved, in some embodiments, by helically winding bead 16 with space between each winding, and then providing the film 18 over the helically wound bead 16 such that at least a first portion of the film 18 (e.g., the portion 18A) is disposed over at least one winding of the bead 16 and a second portion of the film 18 (e.g., the portion 18B) is positioned between the wraps of the bead 16. In this configuration, the smooth bore 20 (and inner surface 22) can be formed by alternating portions of the bead 16 and the film 18.

In some embodiments, the film 18 comprises an elongate strip that is helically wound over the bead 16. The total thickness of the film 18 (i.e., the combined thickness of any and all layers of the film 18, if multiple layers are present) may have a thickness T as shown. In some embodiments, the thickness T is between 0.1 mm and 1 mm. In some embodiments, the thickness Tis between 0.15 mm and 0.4 mm. Other thicknesses T, both larger and smaller than the listed values may also be used.

In an embodiment where the film 18 comprises a plurality of layers of film, where the layers are overlapped, there may be a locally larger thickness than where they are not overlapped. The film 18 can be made from thermoplastic polymer (TPE) material. In some embodiments, the TPE material is polypropylene-based. In another example embodiment, the film can be made of the same material, or the same family of material as the bead 16. In some embodiments, the film 18 may be at least partially optically translucent to enable a user to see condensate or other material within the lumen.

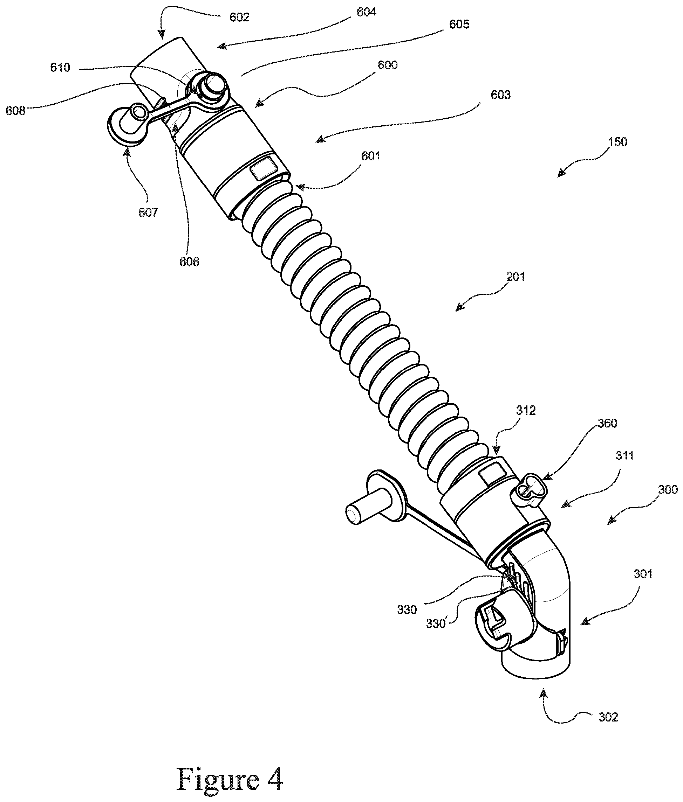

Figure 4 shows an example of a tube 201 having a first connector 300 and a second connector 600. The first connector 300 and the second connector 600 (or further connector) are described below in more detail.

The tube may have an internal diameter of the lumen of about 10 mm to about 25 mm, or about 13 mm to about 20 mm, or about 16 mm to about 18 mm, or about 17.

The tube may have a length of about 1.4 m to about 1.8 m or about 1.5 m to about 1.7 m.

In some embodiments, a lumen of the medical tube may have a smooth bore. Figure 2D shows an example of a tube with an internal smooth bore.

It will be appreciated that such a smooth bore tube may be formed by other manufacturing techniques compared to the first and second elongate member construction as described above of the tube as shown in Figures 2A-2C.

In some embodiments the tube may be a single extrusion.

As shown in Figure 3 the tube 220 may have a smooth bore. The tube 220 may comprise heater wire 221 located within the lumen of the tube.

Figure 3A shows a further example of a tube 222 having a smooth bore. The tube 221 may comprise heater wire 221 located in a tube wall 223 of tube 222. This configuration is similar to that as Figure 2B where the heater wire 221 is spirally wound around a lumen 207 of the tube.

Figure 3B shows a further example of a tube 225 having a smooth bore. The tube 225 may comprise heater wire 221 attached to a tube wall 223 of tube 225. The heater wire 221 may be attached to the inner wall of the tube wall 223 for example by adhesive.

The term "smooth bore" is used herein broadly to describe a substantially smooth inner surface of a lumen extending through the medical tube. In some instances, the term smooth bore may describe a tube that does not have substantial repeating inner surface features (e.g., tubes that have inner surfaces that are not corrugated, wavy, ridged, etc.).

In some instances, the term smooth bore can describe a tube wherein pockets (or cavities, recesses, indentions, etc.) within the tube are minimized, reduced, or eliminated entirely.

One of skill in the art would appreciate that tubes made of multiple elongate members may experience some variation on their inner surfaces. Such minor manufacturing variations, even if repeating, are intended to be encompassed by the term substantially smooth bore.

Medical tubes that include a smooth bore may not substantially disturb (or may decrease disturbances) of a generally laminar flow of gases through the passageway or lumen defined by the smooth bore. Increasing the smoothness of the bore can decrease turbulence and create a more parabolic wave front across the inner wall of the lumen. A smooth bore tube may also provide no pockets in which vapor or gases could be trapped or in which condensate or other liquids might pool, as a corrugated tube would have. The vapor carried by the gases is therefore encouraged to exit the tube and thus be delivered to the patient.

In some embodiments, medical tubes with a smooth bore may advantageously cause the conduit to have a lower resistance to flow (RTF) than a conduit with comparable dimensions having a non-substantially smooth (e.g., corrugated) bore. Thus, in some embodiments, the smooth bore medical tubes described herein may provide improved performance, efficiency, and/or flow as compared to other medical tubes.

It will be appreciated that a tube with an internal smooth bore may have any outer profile (i.e. the outer profile of the tube does not necessarily have to also be smooth).

Figures 4 to 10 show a connector 300 as part of a medical tube assembly 150.

The connector 300 may be located at, and connected to an end of the tube (for example tube 201, or any tube described above).

The connector 300 may provide for a pneumatic connection between the tube and another component.

The connector 300 may provide for electrical connection with wires of the tube (for example wires 215).

The connector 300 may comprise a first portion 301. The first portion 301 may have a first opening 302 configured to receive a flow of gases (for example of flow of gases from a humidification chamber). The first portion 301 may be configured to engage with a humidification chamber and receive gases from the humidification chamber through the first opening 302.

The connector 300 may comprise a second portion 311. The second portion 311 may have a second opening 312. The second portion 311 may be configured to engage with a medical tube (for example tube 201) and provide a flow of gases to the medical tube 201 through the second opening 312.

The connector 300 may comprise a lumen formed by the first portion 301 and the second portion 311. The lumen may provide a gases flow path between the first opening 302 and the second opening 312.

As shown for example in Figures 4 to 5B the first portion 301 may be angled with respect to the second portion 311 to form an elbow.

The elbow configuration of connector 300 may allow for drain back (for example draining in the direction of the chamber) of any condensate in the tube 201 or in the second portion 311 when the connector 300 is connected to a humidification chamber.

The elbow configuration of connector 300 may locate the tube 201 at an angle so that gravity can act on any condensate to return it to the connector 300 and subsequently to an attached humidification chamber.

Drain back of condensate may prevent condensate from reaching a patient, and may increase overall efficiency of the system by redirecting any condensate to the humidification chamber.

The elbow configuration of connector 300 may also act to direct the tube 201 in a direction up and away from the humidifier, so as to keep the area around the humidifier unobstructed by the tube 201. This may allow for easier access and user of a working area

The angle between the first portion 301 and the second portion 311 may be about 90 degrees to about 170 degrees, or about 100 degrees to about 150 degrees, or about 110 to about 140 degrees, or about 135 degrees.

The angle between the first portion 301 and the second portion 311 may be the angle of the internal portion 369 of the elbow.

The angle may be measured between a longitudinal axis of the lumen of the first portion 301 and longitudinal axis of the lumen of the second portion 311 (for example the angle shown by axis 372).

The first portion 301 may provide for a taper fit connection. Taper fit connector may engage with a complimentary taper fit connection of another component such as that of a humidification chamber.

An external surface of the connector may comprise a plurality of gripping features 330. The gripping features 330 may be for grasping by a user.

The gripping features 330 may be configured to present resistance to the fingers of a user during the connection or disconnection of the connector from a gas supply outlet (for example a humidification chamber).

The gripping features 330 may also comprise an indentation. The indentation may be formed by a reduction of the cross-sectional area or profile of the outer surface of the connector 300.

As shown in Figures 4 to 9 the gripping features 330 may comprise at least one pair of ribs or nodes projecting from the external surface of the connector 300. The ribs or nodes of the at least one pair are presented at substantially opposite external surface portions of the connector 300.

The gripping features 330 may comprise three pairs of ribs or nodes, and the ribs or nodes of each pair are presented at substantially opposite external surface portions of the connector 300.

The gripping features 330 may comprise at least one set of ribs 330' (for example as shown in Figure 4), and each rib 330' is oriented such that a longitudinal direction of each rib 330' is substantially non-parallel with a longitudinal axis of the first portion proximate to the first opening 302.

Each rib 330' may be oriented substantially parallel with a longitudinal axis of the second portion proximate to the second opening.

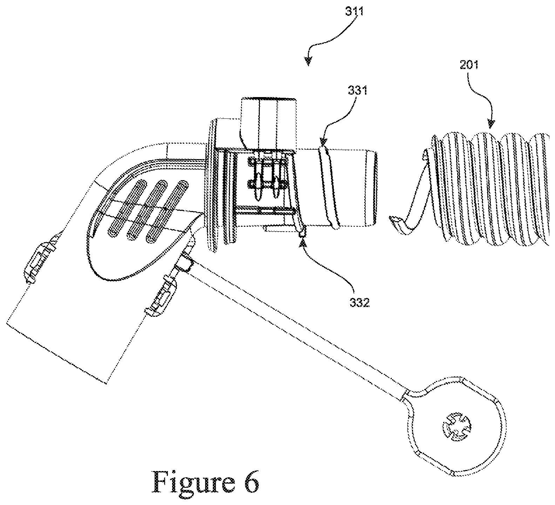

As shown in Figure 6, the second portion 311 may comprises at least one connection feature 331, the connection feature 331 may be configured to engage and retain the medical tube 201.

The connection feature 331 may be a helical rib (for example a thread or screw thread).

In some embodiments, the connection feature 331 may comprise a series of protrusions configured to engage the tube 201.

In some embodiments, the connection feature 331 may comprise a barbed connection.

In some embodiments, the connection feature 331 may comprise any combination of the above described features.

A guide feature or location feature (described in more detail below) may be located at the termination of the connection feature 331, so as to be provided at the end of the medical tube 201 when the medical tube 201 is attached to the connector 300.

The connector 300 may comprise at least one protrusion 332 located at or near one end of the connection feature 331. For example, protrusion 332 can be located at one end of a thread used as a connection feature 331. The protrusion 332 may be configured to provide a stop to engage with the medical tube 201 and prevent further engagement of the medical tube 201 and the thread as a connection feature 331.

The connection between the medical tube 201 and the connector 300 may be overmoulded.

The overmould may assist in retaining the connector 300 on the medical tube.

The overmould may protect the electrical connection from water ingress (for example the connection between the electrical port and the wires 215)

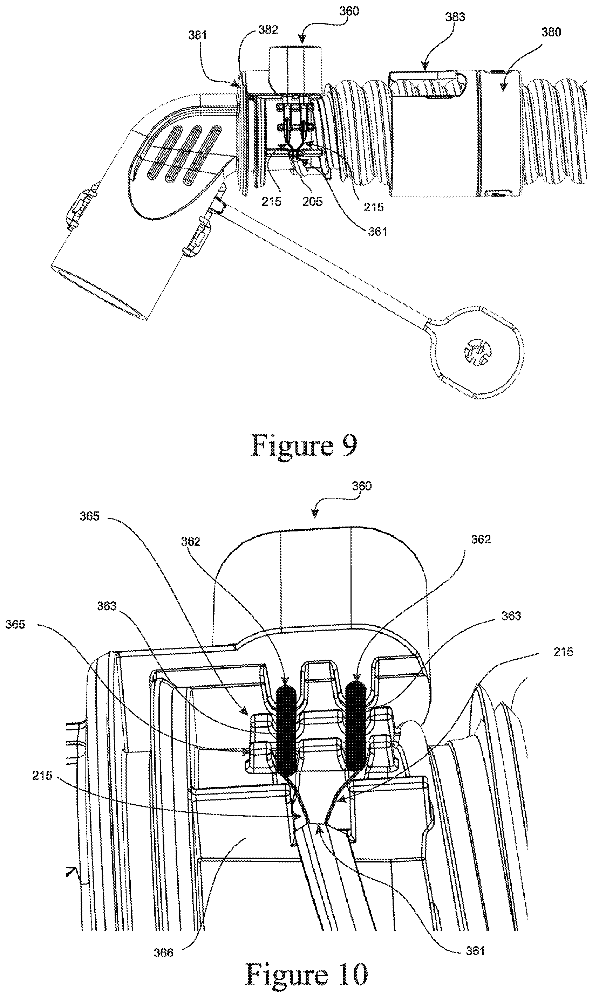

As shown in Figure 9, the connector 300 may comprise a cuff 380 configured to be located over at least part of said second portion 311.

The connector 300 may comprise a terminal collar 381 located away from an end of the second portion 311 (i.e. the second opening). The cuff 380 may be configured to engage a terminal collar 501 when installed.

The terminal collar 381 may be provided around a circumference of the second portion 311.

The connector 300 and cuff 380 may comprise corresponding locking features configured to lock and retain the cuff 380 on the second portion 311.

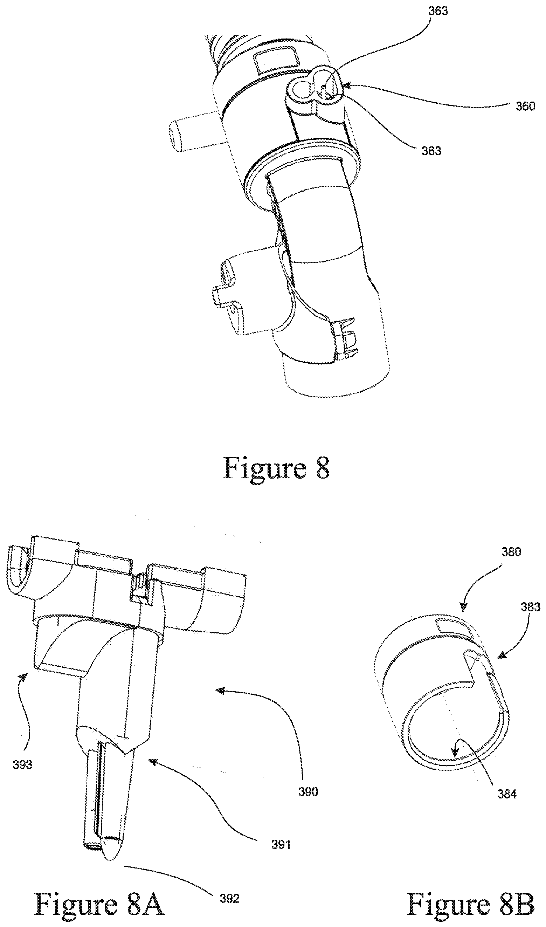

The locking feature may comprises a locking collar 382 and a protrusion 384 (for example as shown by Figure 8B). The protrusion 384 may be configured to engage with the locking collar to engage and connect the connector 300 and the cuff 380.

The locking collar 382 may be provided on an outer surface of the second portion 311 (for example as shown by locking collar 382 in Figure 9), or the protrusion on an inner surface of the cuff 380.

The protrusion 385 may be provided on an outer surface of the second portion 311, or the protrusion 384 on an inner surface of the cuff 380 (as for example shown in Figure 8B).

The locking collar 382 may be located around a circumference of an outer surface of the second portion 311, or an inner surface of the cuff 380.

The protrusion 384 may be located around a circumference of an outer surface of the second portion 311, or an inner surface of the cuff 380 (as shown for example in Figure 8B).

The locking collar may act as a limit surface for an overmould located under the cuff 380 to overmould the interface between the tube 201 and the connector 300.

The cuff may have a cut out 383 located at one end. The cut out 383 may be shaped to accommodate a profile of an electrical port 360.

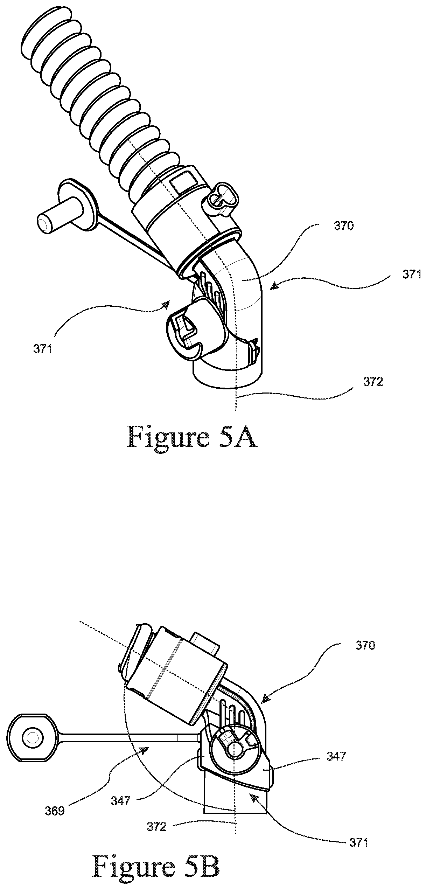

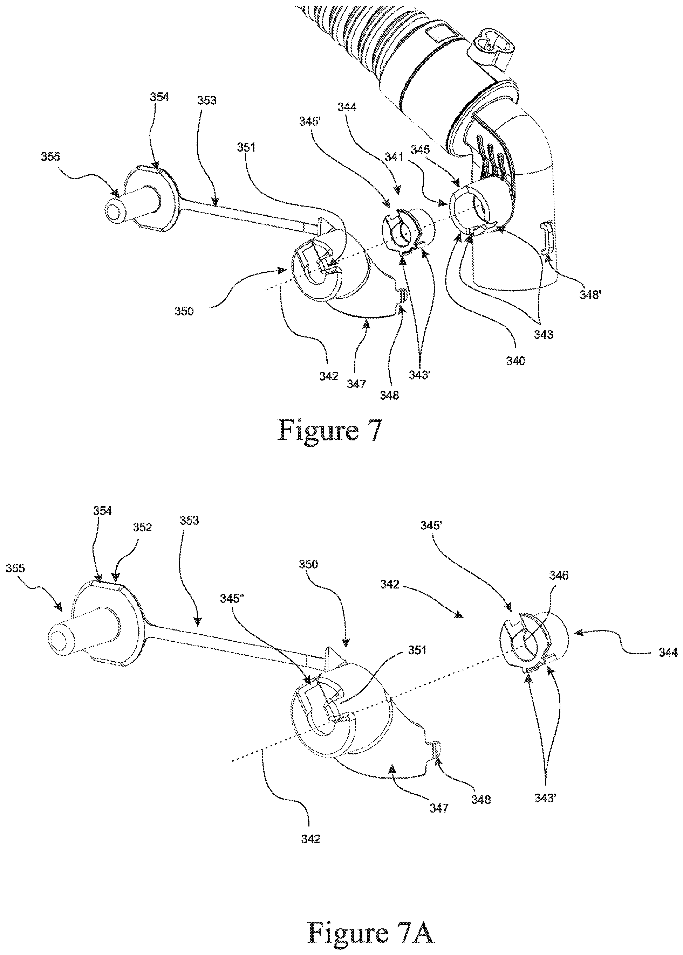

Figures 7 and 7A show connector 300 with a sensor port 340. The sensor port may have a grommet 344, and a jacket 350 with a cover. In use the sensor port 340 houses the grommet 344, and the jacket 350 is provided over the grommet 344 (as shown in Figure 5A and 5B).

As shown in Figure 7 and 7A, the first portion 301 of the connector 300 may comprises a sensor port 340. The sensor port 340 may be configured to receive a sensor.

The sensor port 340 may provide an aperture for the sensor to be inserted so as to be in contact with a flow of gases in the first portion of the connector.

The sensor may be configured to measure flow rate of the flow of gases in the first portion 301 of the connector 300.

The sensor may be configured to measure temperature of the flow of gases in the first portion 301 of the connector 300.

The sensor may be configured to measure humidity of the flow of gases in the first portion 301 of the connector 300.

The sensor may be comprised as part of a probe, the probe for being received by the sensor port 340.

Figure 8A shows an example of a sensor probe 390. The sensor probe may have a portion 391 which extends into the sensor port to position the sensor probe 390 in the flow of gases within either or both connectors 300, 600.

The sensor probe 390 may comprise a sensing portion 392 located at the tip of the probe.

The sensing portion 392 may be positioned within the centre of the lumen of the either or both connectors 300, 600.

The sensing portion 293 may comprise a portion of the sensor which senses a characteristic of the flow of gases.

One or more wires may be connected to the sensor to provide an electrical signal indicative of a characteristic of the flow of gases to one or more controllers.

The sensor port 340 having a longitudinal axis 342, the sensor port 340 allowing communication with an interior of the connector.

The longitudinal axis 342 of the sensor port 340 may be oriented substantially perpendicularly to the flow of gases through the first portion 301 of the connector 300.

The longitudinal axis 342 of the sensor port 340 may substantially intersect a corresponding longitudinal axis of the first portion 301 of the connector.

The sensor port 340 may comprise a substantially cylindrical cross-section.

A terminal surface of the sensor port distal of an outer surface of the first portion 301 may comprise one or more locating features 343. The one or more locating features 343 may comprise one or more notches of the terminal surface of the sensor port 340.

The one or more notches may extend in the direction of the longitudinal axis 342 of the sensor port 340.

The one or more notches may extend towards the centre of the connector.

The locating features 343 may comprise a plurality of notches, the notches spaced apart about a perimeter of the terminal surface and at least one of the plurality of notches configured to mate with a corresponding number of projections of a grommet 344.

The notches as locating features 343 may be substantially V-shaped, or U-Shaped.

The sensor port 340 and/or grommet 344 may comprises a sensor orienting notch 345 for receiving a projection of a or the sensor and rotationally orienting said sensor about the longitudinal axis of the sensor port 340.

The sensor orienting notch may be configured to engage with a corresponding projection 393 located on the sensor (for example as part of sensor probe 390).

The grommet 344 may comprise a sensor locating feature 364 for engaging with a surface of a sensor and locating said sensor at a desired location on or parallel to the longitudinal axis of the sensor port 340.

The sensor locating feature 346 may comprise a radially extending lip spaced away from the terminal surface of the grommet and projecting inwardly towards the longitudinal axis of the sensor port 346.

The sensor locating feature 364 may be formed by a decrease in the internal diameter of the grommet 344.

In some embodiments the grommet seals with a surface of the sensor.

As shown in Figures 7 and 7a, the connector 300 may comprise a jacket 350 for the sensor port 340, the jacket 350 is for fitment at least partially about the sensor port 340 and/or the grommet 344.

The jacket 350 may comprise a sensor orienting notch 345" for receiving a projection of a said sensor and rotationally orienting said sensor about the longitudinal axis of the sensor port 340 and/or grommet 344, and relative to the jacket 350.

The sensor orienting notch 345" of the jacket 350 may be configured to engage with the sensor orienting notch 345' of the grommet 344 and the sensor orienting notch 345 of sensor port 340 to allow for the mating of the jacket 350, sensor port 340 and grommet 344 in only one relative rotational position.

The jacket may comprise a sensor depth locating feature 351 for engaging with a surface of the sensor. The sensor depth locating feature 351 may locate the sensor at a desired distance within the first portion 301 of the connector 300.

The sensor depth locating feature 351 may comprise a projection as an upstand.

The sensor depth locating feature 351 may locate the sensor at a desired location on or parallel to the longitudinal axis of the sensor port 340.

The sensor depth locating feature 351 may provide a limit to the passage of the sensor within the sensor port 340.

As shown in Figure 7A, the jacket 350 may comprise two securing members 347 for securing the jacket to either or both of the sensor port 340 and the first portion.

The securing members may 347 secure the jacket 350 to the first portion 301.

The securing members 347 may at least partially encircle or cover an outer surface of the first portion 301 from which the sensor port depends 340.

The securing members 347 may at least partially encircle and are configured to frictionally engage with the outer surface of the first portion 301 such as to secure the jacket 350 to the first portion 301.

The securing members 347 comprise locking features 348 to engage with corresponding locking features 348' of the respective first portion 301.

The locking features 348' of the first portion may be located on substantially opposite sides of said first portion 301.

The locking features 348' of the first portion are spaced apart from each other along the lumen and/or a longitudinal axis of the first portion.

The locking features of the first portion are aligned substantially parallel to a longitudinal axis of the second portion.

The connector 300 may comprises a cover 352 for coupling with the sensor port 351 and for sealing the sensor port 340, and/or grommet 344, the cover comprising a projection 355 for location within and a sealing of the sensor port 340 and/or grommet 344.

In some embodiments, the jacket 350 may comprise said cover 352. The cover 352 may be connected to the jacket 350 by a tether 353.

When the cover 352 is not coupled with the sensor port 340 the tether 353 may be configured to be located away from the sensor port 340.

When the cover 352 is not coupled with the sensor port 340 the tether 353 may be configured to extend substantially perpendicularly to the longitudinal axis 342 of the sensor port 340.

The cover 352 may comprises a gripping feature 354 to aid in gripping of the cover by a user. The gripping feature may be an outwardly extending collar. In some embodiments, when the sensor port 340 is sealed by the projection 355.

When the sensor port 340 is sealed by the projection 355 the gripping feature may be presented away from the jacket 350 such that the gripping feature 354 may be grasped by a user.

The gripping feature 354 may not extend laterally past an extent of a portion of the jacket fitted within the sensor port 340.

The sensor depth locating feature 351 may be configured to act as an upstand and engage with the gripping feature 354 and provide a limit to the passage of the projection within the sensor port 340.

As illustrated with respect to Figure 8 to 10 the connector 300 may comprise at least one electrical port 360. The electrical port may be configured to provide an electrical connection with one or more wires 215 of the tube.

The electrical port may receive another electrical port. The electrical port may comprise one or more conductors (for example a pin) for engaging with a corresponding conductor of another electrical port.

In some embodiments the electrical port comprises two pins.

The wire 215 (for example heater wire) may be spirally wound along at least a portion of the length of the medical tube 201 (for example as shown in Figures 2B, 3A and 9 and 10.

The electrical port 360 may comprise at one least connection point 362. The connection point 362 may be configured to provide for connection of the electrical port to the at least one wire 215.

As shown in Figure 4 the electrical port 360 may be offset from a longitudinal central axis of the second portion 311 of the connector 300. This positioning allows for alignment of the electrical port 360 with an outer surface of the connector 300.

In some embodiments, the electrical port 360 may be aligned tangential aligned with an outer surface of the connector 300.

This positioning allows for alignment of the electrical port 360 with an outer surface of the connector 300.

The outer surface of the connector 300 may be the surface located between the end of the tube, or where the wire exits the tube wall, and an electrical connector.

Aligning the outer surface of the connector 300 and the electrical port 360 allows for alignment between the wire 215 and the connector 300 when the tube 201 is engaged with the connector 300.

In some embodiments, a termination point 361 of the wire 215 from the tube (for example where the wire exits the tube wall) may be aligned with said electrical port.

The electrical port 360 may comprise at least one connection point 362. The connection point 362 acts as an interface between the electrical port 360 and the wire 215 to provide for connection of the electrical port to the at least one wire.

The connection point 362 may be a aperture into the electrical port 360.

The connection point 362 may be aligned with an outer surface of the connector 360.

The connection point 362 of the electrical port may be aligned with a surface tangential to an outer surface of the connector 360.

In some embodiments, a termination point 361 of the wire from the tube (for example where the wire exits the tube wall) may be aligned with the connection point 362. This aligns the wire 215 as it exits the tube wall with the connection point 362.

The alignment of the wire and the connector 360 and/or the connection point 362 allows for a direct pathway to be provided from a termination point of the wire from the tube wall to the connection point.

Alignment of the wire and the connector 360 and/or the connection point 362 leads to ease of manufacture as when the tube 201 is attached to the connector 205 the wires 215 directly align with the connector. No repositioning or realigning of wires are required as when the tube 201 is installed the termination point of the wire from the tube wall and the electrical port and/or connection point 362 are already aligned.

The electrical port 360 may comprise at least one pin 363 configured to be in electrical communication with said at least one wire 215. The pin 363 may be for example a crimp pin to connect to the end of the wire 216.

The electrical port 360 may comprise two pins 363 configured to be in electrical communication with said at least one wire 215.

The electrical port 360 and/or the connection point 362 of the electrical port 300 may be configured to receive at least one wire 215 of the tube wall, or the pin (for example a crimp pin) connected to said at least one wire 215. The pin 363 may engage with the connection point (for example by extending through the connection point to form electrical connection pin 363).

The pin 363 may provide for electrical connection to another electrical port (for example a plug) when engaged with the electrical port 360.

The electrical port 360 may be formed integrally with the connector.

The electrical port 360 as shown in Figure 8 may be in the shape of a cloverleaf.

The electrical port 360 may comprise a first portion, a second portion and a third portion. The first portion and second portion comprise the at least one pin configured to be in electrical communication with said at least one wire. The third portion is configured to receive a locating portion of a corresponding electrical port.

The tube 201 may be provided to the second portion 311 of the connector 300 and engaged with said connection features 331, such that the wires 215 of the tube 201 extending from the tube wall are aligned with said electrical port 360 and/or the connection point 363.

The connector 300 may comprise at least one guide feature 365 configured to direct the at least one wire 215 from the tube wall of the tube 201, and/or a pin connected to said at least one wire 215, to the electrical port 360 and/or the connection point 264 of the electrical port 360.

The at least one guide feature 365 may locate said wire 215 and/or pin 364 in a predetermined location.

The predetermined location of the wire 215 and/or pin 364 may be aligned with the electrical port 360 and/or the connection point 364 of the electrical port 360.

The at least one guide feature 365 may be provided by an outer surface of the connector 300.

The at least one guide feature 365 may be formed at least in part by one or more recesses or protrusions.

The at least one guide feature 365 may be provided on the second portion 311 of the connector 300.

The at least one guide feature 365 may comprise at least one rib, wherein the rib is oriented parallel or along a longitudinal axis of the second portion, or a longitudinal axis of the medical tube 201.

The at least one guide feature may comprise a rib having one or more recesses configured to locate at least one wire 215 and/or said at least one pin 264 within the recess.

Where the tube 201 is the tube of the type as shown in Figures 2A-2C, having an elongate member 205 with an embedded wire, the connector may comprise at least one location feature 366. The location feature 366 may be configured to locate the elongate member (for example the second elongate member 205) from the end of the tube into a predetermined location.

The predetermined location may align the second elongate member 205 with the electrical port 360 and/or the connection point 364 of the electrical port 360.

Alignment of the second elongate member 205 with the electrical port 360 and/or the connection point 364 allows for corresponding alignment of the termination point 261 (where the wires 215 exit the tube).

The predetermined location allows for the wires 215 from the second elongate member 205 to be provided to corresponding guide features365.

The location features 366 may comprise one or more ribs, and/or recesses, oriented parallel or along a longitudinal axis of the second portion 311, or a longitudinal axis of the medical tube 201.

The location features 366 may comprise a rib having a recess, the recess configured to locate said second elongate member at said predetermined location.

In some embodiments, the location feature may comprise a hook, loop or a clip.

As discussed above easy access to both the sensor port 340 and the electrical port 360 is required so as to allow for easy connection and disconnection of the relevant probes and connectors to the connector 300.

The electrical port 360 being on as described above (for example on the top of the connector 300) may also reduce the likelihood of any cabling connected to the electrical port 360 interfering with other cabling of the system (for example cabling associated with the sensor port 340 and /or sensor probe 390).

The direction of the electrical port 360 (for example up and away from the humidifier) may also direct any cabling away from the humidifier and/or a working area of the humidifier.

If for example the sensor port 340 were to be located on the same portion of the connector then it may be difficult for a clinician to easily independently connect and disconnect relevant probes and connectors to the sensor port 340 and the electrical port 360.

As shown in Figures 5A and 5B the sensor port 340 may be located on a side 371 of the first portion 301 of the connector 300 and the electrical port 360 is located on the top of the second portion 311 of the connector 300.

The top of the second portion 311 of the connector 300 may be the external portion 370 of the elbow (i.e. the outer portion of the elbow).

The side(s) 371 of the first portion 301 of the connector 300 may be either side of the elbow.

The sensor port may offset about 120 to about 75 degrees, or about 110 to about 80 degrees, or about 90 degrees, about a longitudinal axis 372 of the lumen of the connector 300 relative to electrical port 360.

The connector 300 may define internal and external elbow surfaces, and wherein the electrical port is located on the external elbow surface 370.

The sensor port 340 may be oriented perpendicular to the first portion 301.

The sensor port 340 may be oriented perpendicular to a longitudinal axis of the first portion 301 (for example the portion of axis 372 in the first portion).

The electrical port 360 may be oriented along an axis parallel to an axis substantially perpendicular to the second portion.

The electrical port 360 may be oriented along an axis parallel substantially perpendicular to a longitudinal axis of the second portion.

The electrical port 360 may be offset from the centre of the second portion 311 of the connector 300.

The electrical port 360 may be oriented tangentially to an outer surface of the connector 300.

The electrical port 360 may be located perpendicular to the sensor port 340.

An axis passing through the electrical port 360 may be located perpendicular to an axis passing through the sensor port 340

The electrical port 360 may extend in a direction upward and away from the humidifier when the connector 300 is connected to a humidification chamber.

The sensor port 340 may extend in a direction away from the humidifier when the connector 300 is connected to a humidification chamber.

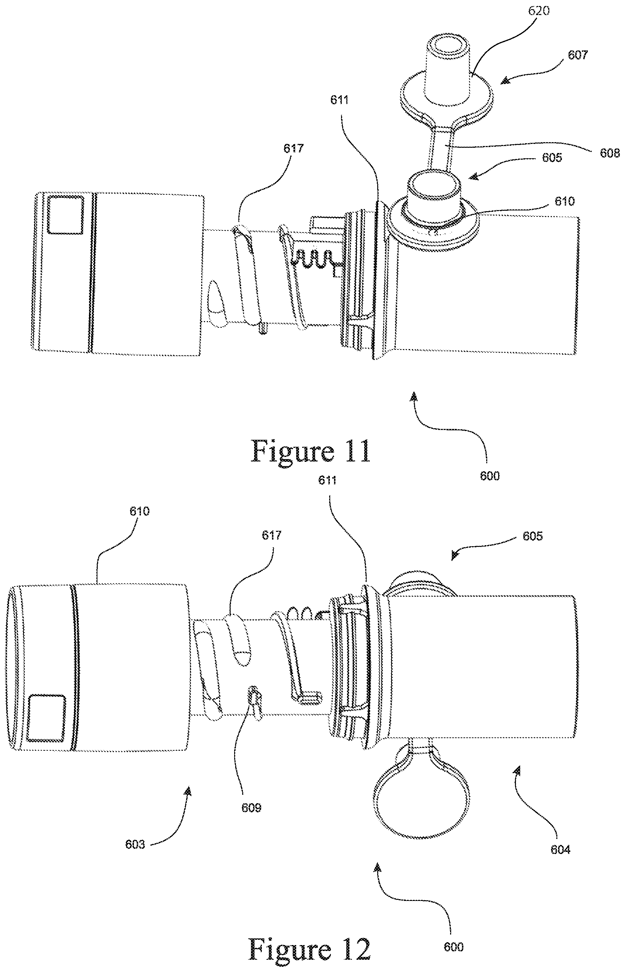

Figures 6, 11, 12 and 13 show another connector 600 as part of a medical tube assembly.

The connector 600 may comprise a first end 604 having a first opening 602.

The connector 600 may comprise a second end 603 having a second opening 601.

The connector 600 may be configured for connection with a medical tube at a second end 603.

The first end 604 of the connector may be configured for engagement with a patient interface, and/or a patient interface extension tube.

The first opening 602 may be configured to provide a flow of gases.

The connector 600 may receive a flow of gases from the medical tube 201 through the second opening 602.

The connector 600 may comprise lumen formed by the connector 600, the lumen providing a gases flow path between the second opening 601 and the first opening 602.

The connector 600 may comprise at least one sensor port 605. The sensor port 605 may have any of the features of the sensor port 340 as described above (for example the grommet 344 and/or jacket 350.)

The sensor port 605 may extend outwardly from the connector 600.

The sensor port 605 may comprise at least one cover 607.

The cover 607 may be attached to the connector 600 about the sensor port 605.

The cover 607 may be retained around the sensor port 605 by one or more retention features 610.

The retention feature 610 may comprise one or more projections.

The cover 607 may comprise a cap 620 connected to the cover by a tether 608.

The connector 600 may comprise at least one connection feature 617 configured to allow for connection with the medical tube.

The connection feature 617 may be a helical rib (for example a thread or screw thread).

In some embodiments, the connection feature 617 may comprise a series of protrusions configured to engage the tube.

In some embodiments, the connection feature 617 may comprise a barbed connection.

In some embodiments, the connection feature 617 may comprise any combination of the above described features.

The connector 600 may comprise at least one protrusion 609 located at or near the end of the thread, the protrusion 609 configured to provide a stop to engage with the medical tube and prevent further engagement of the medical tube and the thread.

The connector may comprise a cuff 610 configured to be located over at least part of said second end.

The connector 600 may comprise a terminal collar 611, the terminal collar 611 being located away from an end of the second end (i.e. the second opening) the cuff 610 configured to engage said terminal collar 611 when installed.

The terminal collar 611 may be provided around a circumference of the second end.

The connector 600 and cuff 610 may comprise corresponding locking feature(s) configured to lock and retain the cuff on the second portion.

The locking feature may comprise a locking collar and a protrusion.

The protrusion may be configured to engage with the locking collar to engage and connect the connector 600 and the cuff 610.

The locking collar may be provided on an outer surface of the second end, or the protrusion on an inner surface of the cuff 610.

The protrusion may be provided on an outer surface of the second end, or the protrusion on an inner surface of the cuff 610.

The locking collar may be located around a circumference of an outer surface of the second end, or an inner surface of the cuff 610.

The protrusion may be located around a circumference of an outer surface of the second end, or an inner surface of the cuff 610.

The locking collar may act as a limit surface for an overmould located under the cuff 610 to overmould the interface between the tube and the connector.

The cuff 610 and the connector 600 comprise corresponding alignment features.

The alignment features 612, 613 comprise at least one protrusion 612 and a corresponding at least one recess 613.

The alignment features 612, 613 may be configured to prevent relative rotation between the cuff and the connector.

The at least one protrusion 612 comprises one or more teeth.

The cuff may comprise said at least one recess 613, and the connector comprises said at least one protrusion 612.

In some embodiments the connector may comprise said at least one recess 613, and the connector may comprise said at least one protrusion.

The at least one recess 613 may located on an internal surface of the cuff 610.

The at least one recess 613 may comprise a plurality of recesses, such that the cuff 610 may engage with the protrusions 612 in a plurality of orientations.

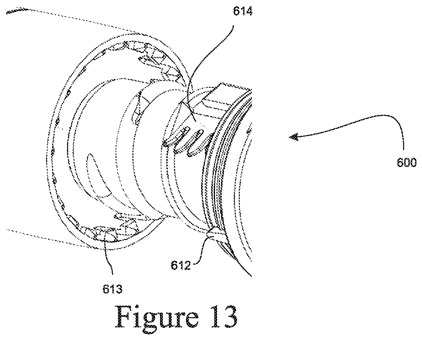

As shown in Figure 13, the at least one recess 613 may located around the entire circumference of the internal surface of the cuff 610.

The at least one recess 613 may be patterned about the circumference of the internal surface of the cuff 610.

The at least one protrusion 612 may be located on the connector.

The at least one protrusion 612 may be located on a or the terminal collar 611 or a or the locking collar of connector 600.

The connector may comprise a wire termination feature 614. The wire termination feature 614 may be configured to provide for termination of a or the wire of the tube at the connector.

The wire termination feature 614 may comprise one or more projections extending in a direction away from the connector.

The first end 604 of the connector 600 may provide for a taper fit connection.

## Claims

1. A medical tube assembly (150), the medical tube assembly (150) comprising:
a medical tube (201), the medical tube (201) having at least one wire located in a tube wall (223) of the medical tube (201), wherein the at least one wire is helically wound,
a connector, the connector located at an end of the medical tube (201),
wherein the connector comprises at least one electrical port (360), the electrical port (360) extending outwardly from the connector, the electrical port (360) configured to provide an electrical connection with the at least one wire, preferably the electrical port (360) comprises at least one connection point, the connection point configured to provide for connection of the electrical port (360) to the at least one wire,
wherein the connector comprises at least one guide feature (365) configured to direct the at least one wire from the tube wall (223) of the tube (201), and/or a pin connected to the at least one wire, to the electrical port (360) and/or the connection point of the electrical port (360),
wherein the electrical port (360) is located tangentially with respect to an outer surface of the connector,
wherein the at least one guide feature (365) locates the at least one wire and/or the pin connected to the at least one wire in a predetermined location, wherein the predetermined location is aligned with the electrical port (360) and/or the connection point of the electrical port (360).

2. The medical tube assembly (150) of claim 1, wherein the electrical port (360) is configured to extend in a direction upward and away from a humidifier when the connector is connected to a humidification chamber.

3. The medical tube assembly (150) of claim 1 or claim 2, wherein the electrical port (360) is configured to extend in a direction away from a or the humidifier when the connector is connected to a humidification chamber.

4. The medical tube assembly (150) of any one of claims 1 to 3, comprising a first portion (301) having a first opening (302) configured to receive a flow of gases, and a second portion (311) having a second opening (312), the second portion (311) configured to engage with the medical tube (201) and provide a flow of gases to the medical tube (201) through the second opening (312), wherein the first portion (301) comprises a sensor port, the sensor port configured to receive a sensor.

5. The medical tube assembly (150) of claim 4, wherein the second portion (311) comprises the electrical port (360) and the at least one guide feature (365).

6. The medical tube assembly (150) of claim 5, wherein the first portion (301) is angled with respect to the second portion (311) to form an elbow.

7. The medical tube assembly (150) of claim 6, wherein the angle between the first portion (301) and the second portion (311) is about 90 degrees to about 170 degrees, or about 100 degrees to about 150 degrees, or about 110 to about 140 degrees, or about 135 degrees.

8. The medical tube assembly (150) of any of claims 1 to 7, wherein the tube (201) comprises a lumen extending from a first end of the tube (201) to a second end of the tube (201), a first elongate member (203) comprising a hollow body spirally wound to form at least in part the lumen, a second elongate member (205) spirally wound and joined between adjacent turns of the first elongate member (203).

9. The medical tube assembly (150) of claim 8, wherein the at least one wire is located within the second elongate member (205).

10. The medical tube assembly (150) of claim 9, wherein the at least one wire comprises at least one heater wire.

11. The medical tube assembly (150) of claim 9 or claim 10, wherein the at least one wire comprises at least one sensor wire.

12. The medical tube assembly (150) of any of claims 1 to 11, wherein the connection between the medical tube (201) and the connector is overmoulded.

13. The medical tube assembly (150) of any of claims 1 to 12, wherein at least one guide feature (365) is formed at least in part by one or more recesses and/or protrusions.

14. The medical tube assembly (150) of any of claims 1 to 13, at least one guide feature (365) comprises at least one rib, wherein the rib is oriented parallel or along a longitudinal axis of the second portion (311), or a longitudinal axis of the medical tube (201).

15. The medical tube assembly (150) of any preceding claims, wherein the at least one guide feature (365) comprises a rib having a recess configured to locate at least one wire and/or the at least one pin within the recess.

## Patentansprüche

1. Medizinische Schlauchanordnung (150), die medizinische Schlauchanordnung (150) umfassend:
einen medizinischen Schlauch (201), wobei der medizinische Schlauch (201) mindestens einen in einer Schlauchwand (223) des medizinischen Schlauchs (201) angeordneten Draht aufweist, wobei der mindestens eine Draht schraubenförmig gewickelt ist,
eine Steckverbindung, wobei die Steckverbindung an einem Ende des medizinischen Schlauchs (201) angeordnet ist,
wobei die Steckverbindung mindestens einen elektrischen Anschluss (360) umfasst, der sich von der Steckverbindung nach außen erstreckt, wobei der elektrische Anschluss (360) so konfiguriert ist, dass er eine elektrische Verbindung mit dem mindestens einen Draht herstellt, wobei der elektrische Anschluss (360) vorzugsweise mindestens einen Verbindungspunkt umfasst, der so konfiguriert ist, dass er eine Verbindung des elektrischen Anschlusses (360) mit dem mindestens einen Draht ermöglicht,
wobei die Steckverbindung mindestens ein Führungselement (365) umfasst, das so konfiguriert ist, dass es das mindestens eine Kabel von der Schlauchwand (223) des Schlauchs (201), und/oder einen Stift, der mit dem mindestens einen Kabel verbunden ist, zu dem elektrischen Anschluss (360) und/oder den Anschlusspunkt des elektrischen Anschlusses (360) führt,
wobei der elektrische Anschluss (360) tangential im Verhältnis zu einer Außenfläche der Steckverbindung positioniert ist,
wobei das mindestens eine Führungselement (365) den mindestens einen Draht und/oder den mit dem mindestens einen Draht verbundenen Stift an einer vorbestimmten Stelle positioniert, wobei die vorbestimmte Stelle an dem elektrischen Anschluss (360) und/oder dem Verbindungspunkt des elektrischen Anschlusses (360) ausgerichtet ist.

2. Medizinische Schlauchanordnung (150) nach Anspruch 1, wobei der elektrische Anschluss (360) so konfiguriert ist, dass er sich in einer Richtung nach oben und von einem Befeuchter weg erstreckt, wenn die Steckverbindung mit einer Befeuchtungskammer verbunden ist.

3. Medizinische Schlauchanordnung (150) nach Anspruch 1 oder Anspruch 2, wobei der elektrische Anschluss (360) so konfiguriert ist, dass er sich in einer Richtung weg von einem Befeuchter erstreckt, wenn die Steckverbindung mit einer Befeuchtungskammer verbunden ist.

4. Medizinische Schlauchanordnung (150) nach einem der Ansprüche 1 bis 3, umfassend einen ersten Abschnitt (301) mit einer ersten Öffnung (302), die so konfiguriert ist, dass sie einen Gasstrom aufnimmt, und einen zweiten Abschnitt (311) mit einer zweiten Öffnung (312), wobei der zweite Abschnitt (311) so konfiguriert ist, dass er mit dem medizinischen Schlauch (201) in Eingriff tritt und dem medizinischen Schlauch (201) durch die zweite Öffnung (312) einen Gasstrom zuführt, wobei der erste Abschnitt (301) einen Sensoranschluss umfasst, der so konfiguriert ist, dass er einen Sensor aufnimmt.

5. Medizinische Schlauchanordnung (150) nach Anspruch 4, wobei der zweite Abschnitt (311) den elektrischen Anschluss (360) und das mindestens eine Führungselement (365) umfasst.

6. Medizinische Schlauchanordnung (150) nach Anspruch 5, wobei der erste Abschnitt (301) im Verhältnis zu dem zweiten Abschnitt (311) abgewinkelt ist, um ein Winkelstück zu formen.

7. Medizinische Schlauchanordnung (150) nach Anspruch 6, wobei der Winkel zwischen dem ersten Abschnitt (301) und dem zweiten Abschnitt (311) etwa 90 Grad bis etwa 170 Grad oder etwa 100 Grad bis etwa 150 Grad oder etwa 110 bis etwa 140 Grad oder etwa 135 Grad ist.

8. Medizinische Schlauchanordnung (150) nach einem der Ansprüche 1 bis 7, wobei der Schlauch (201) ein Lumen umfasst, das sich von einem ersten Ende des Schlauchs (201) zu einem zweiten Ende des Schlauchs (201) erstreckt, ein erstes längliches Element (203), das einen Hohlkörper umfasst, der spiralförmig gewickelt ist, um mindestens teilweise das Lumen zu bilden, und ein zweites längliches Element (205), das spiralförmig gewickelt und zwischen benachbarten Windungen des ersten länglichen Elements (203) verbunden ist.

9. Medizinische Schlauchanordnung (150) nach Anspruch 8, wobei der mindestens eine Draht innerhalb des zweiten länglichen Elements (205) angeordnet ist.

10. Medizinische Schlauchanordnung (150) nach Anspruch 9, wobei der mindestens eine Draht mindestens einen Heizdraht umfasst.

11. Medizinische Schlauchanordnung (150) nach Anspruch 9 oder Anspruch 10, wobei der mindestens eine Draht mindestens einen Sensordraht umfasst.

12. Medizinische Schlauchanordnung (150) nach einem der Ansprüche 1 bis 11, wobei die Verbindung zwischen dem medizinischen Schlauch (201) und der Steckverbindung umspritzt ist.

13. Medizinische Schlauchanordnung (150) nach einem der Ansprüche 1 bis 12, wobei das mindestens eine Führungselement (365) mindestens teilweise durch eine oder mehrere Vertiefungen und/oder Vorsprünge gebildet ist.

14. Medizinische Schlauchanordnung (150) nach einem der Ansprüche 1 bis 13, wobei das mindestens eine Führungselement (365) mindestens eine Rippe umfasst, wobei die Rippe parallel zu oder entlang einer Längsachse des zweiten Abschnitts (311) oder einer Längsachse des medizinischen Schlauchs (201) ausgerichtet ist.

15. Medizinische Schlauchanordnung (150) nach einem der vorhergehenden Ansprüche, wobei das mindestens eine Führungselement (365) eine Rippe mit einer Vertiefung umfasst, die so konfiguriert ist, dass sie mindestens einen Draht und/oder den mindestens einen Stift innerhalb der Vertiefung positioniert.

## Revendications

1. Ensemble de tubes médicaux (150), l'ensemble de tubes médicaux (150) comprenant :
un tube médical (201), le tube médical (201) présentant au moins un fil situé dans une paroi tubulaire (223) du tube médical (201), dans lequel ledit au moins un fil est enroulé de manière hélicoïdale,
un connecteur, le connecteur situé à une extrémité du tube médical (201),
dans lequel le connecteur comprend au moins un port électrique (360), le port électrique (360) s'étendant vers l'extérieur à partir du connecteur, le port électrique (360) conçu pour fournir une connexion électrique avec au moins un fil, de préférence le port électrique (360) comprend au moins un point de connexion, le point de connexion conçu pour fournir la connexion du port électrique (360) à au moins un fil,
dans lequel le raccordement comprend au moins un élément de guidage (365) conçue pour diriger ledit au moins un fil depuis la paroi tubulaire (223) du tube (201), et/ou une broche connectée audit au moins un fil, vers le port électrique (360) et/ou le point de connexion du port électrique (360),
dans lequel le port électrique (360) est situé de façon tangentielle par rapport à une surface extérieure du connecteur,
dans lequel ledit au moins un élément de guidage (365) situe ledit au moins un fil et/ou la broche connectée audit au moins un fil à un emplacement prédéfini, dans lequel l'emplacement prédéfini est aligné avec le port électrique (360) et/ou le point de connexion du port électrique (360).

2. Ensemble de tubes médicaux (150) selon la revendication 1, dans lequel le port électrique (360) est conçu pour s'étendre dans une direction vers le haut et à l'écart d'un humidificateur lorsque le connecteur est connecté à une chambre d'humidification.

3. Ensemble de tubes médicaux (150) selon la revendication 1 ou la revendication 2, dans lequel le port électrique (360) est conçu pour s'étendre dans une direction à l'écart d'un ou de l'humidificateur lorsque le connecteur est connecté à une chambre d'humidification.

4. Ensemble de tubes médicaux (150) selon l'une quelconque des revendications 1 à 3, comprenant une première partie (301) présentant une première ouverture (302) conçue pour recevoir un flux gazeux, et une seconde partie (311) présentant une seconde ouverture (312), la seconde partie (311) conçue pour s'engager dans le tube médical (201) et fournir un flux gazeux au tube médical (201) à travers la seconde ouverture (312), dans lequel la première partie (301) comprend un port de capteur, le port de capteur conçu pour recevoir un capteur.

5. Ensemble de tubes médicaux (150) selon la revendication 4, dans lequel la seconde partie (311) comprend le port électrique (360) et ledit au moins un élément de guidage (365).

6. Ensemble de tubes médicaux (150) selon la revendication 5, dans lequel la première partie (301) est inclinée par rapport à la seconde partie (311) pour former un coude.

7. Ensemble de tubes médicaux (150) selon la revendication 6, dans lequel l'angle entre la première partie (301) et la seconde partie (311) est d'environ 90 degrés à environ 170 degrés, ou d'environ 100 degrés à environ 150 degrés, ou d'environ 110 à environ 140 degrés, ou d'environ 135 degrés.

8. Ensemble de tubes médicaux (150) selon l'une quelconque des revendications 1 à 7, dans lequel le tube (201) comprend une lumière s'étendant d'une première extrémité du tube (201) à une seconde extrémité du tube (201), un premier élément allongé (203) comprenant un corps creux enroulé en spirale pour former au moins en partie la lumière, un second élément allongé (205) enroulé en spirale et relié entre des tours adjacents du premier élément allongé (203).

9. Ensemble de tubes médicaux (150) selon la revendication 8, dans lequel ledit au moins un fil est situé à l'intérieur du second élément allongé (205).

10. Ensemble de tubes médicaux (150) selon la revendication 9, dans lequel ledit au moins un fil comprend au moins un fil de chauffage.

11. Ensemble de tubes médicaux (150) selon la revendication 9 ou 10, dans lequel ledit au moins un fil comprend au moins un fil de capteur.

12. Ensemble de tubes médicaux (150) selon l'une quelconque des revendications 1 à 11, dans lequel la connexion entre le tube médical (201) et le connecteur est surmoulée.

13. Ensemble de tubes médicaux (150) selon l'une quelconque des revendications 1 à 12, dans lequel au moins un élément de guidage (365) est formé au moins en partie par un ou plusieurs évidements et/ou saillies.

14. Ensemble de tubes médicaux (150) selon l'une quelconque des revendications 1 à 13, au moins un élément de guidage (365), comprend au moins une nervure, dans lequel la nervure est orientée parallèlement ou le long d'un axe longitudinal de la seconde partie (311), ou un axe longitudinal du tube médical (201).

15. Ensemble de tubes médicaux (150) selon l'une quelconque des revendications précédentes, dans lequel ledit au moins un élément de guidage (365) comprend une nervure présentant un évidement conçu pour localiser au moins un fil et/ou ladite au moins une broche dans l' évidement.
